# EUROPÄISCHE PATENTANMELDUNG

(11) **EP 4 066 801 A1**
(43) Veröffentlichungstag der Anmeldung: **05.10.2022**
(21) Anmeldenummer: 22165474.2
(22) Anmeldetag: 30.03.2022
(51) Int. Cl.: A61F 13/49, A61F 13/532, A61F 13/534

(54) **ABSORBIERENDER KERN FÜR EINEN HYGIENEARTIKEL**

(30) Priorität: 30.03.2021 DE 102021108110
(71) Anmelder: LK Mahnke GmbH & Co., KG, 45479 Mühlheim and der Ruhr (DE)
(72) Erfinder: HAAS, Alexandra, 42781 Haan (DE); PAZ, Rui Miguel, 47800 Krefeld (DE)
(74) Vertreter: Gottschald Patentanwälte Partnerschaft mbB

(57) **Zusammenfassung**

Die Erfindung betrifft einen absorbierenden Kern für einen Hygieneartikel (1), insbesondere für eine Windel, wobei der absorbierende Kern (4) eine Längsrichtung (L), eine Breitenrichtung (B), ein körperseitiges Deckblatt (8), ein körperfernes Rückenblatt (9) und eine zwischen dem Deckblatt (8) und dem Rückenblatt (9) angeordnete Schicht (10) mit Saugmaterial, vorzugsweise umfassend Superabsorber, aufweist, wobei die Schicht (10) mit Saugmaterial Saugkanäle (23) mit einer Flüssigkeitsleitcharakteristik aufweist, wobei die Schicht (10) mit Saugmaterial durch Bahnen (16, 17, 18, 18a), insbesondere Kanäle, mit einer abweichenden Flüssigkeitsleitcharakteristik in die Saugkanäle (23) geteilt ist, wobei die Schicht (10) mit Saugmaterial in einem ersten Abschnitt (24) entlang der Längsrichtung (L) mindestens drei Saugkanäle (23) aufweist und in einem zweiten Abschnitt (25) weniger Saugkanäle (23) als im ersten Abschnitt (24) jedoch mindestens zwei Saugkanäle (23) aufweist. Es wird vorgeschlagen, dass der absorbierende Kern (4), insbesondere die Schicht (10) mit Saugmaterial, in Längsrichtung (L) hintereinander angeordnete Zonen (20) mit unterschiedlicher spezifischer Kapazität aufweist und dass der absorbierende Kern (4) in dem ersten Abschnitt (24) und in dem zweiten Abschnitt (25) zumindest teilweise unterschiedliche spezifische Kapazitäten aufweist.

## Beschreibung

Die Erfindung betrifft einen absorbierenden Kern für einen Hygieneartikel gemäß dem Oberbegriff von Anspruch 1, einen Hygieneartikel gemäß Anspruch 14 sowie ein Herstellungsverfahren für einen absorbierenden Kern gemäß dem Oberbegriff von Anspruch 15.

Derartige absorbierende Kerne werden in unterschiedlichen Hygieneartikeln verwendet. Diese Hygieneartikel können Windeln, insbesondere Babywindeln, aber auch Erwachsenenwindeln und Inkontinenzwindeln sein. Die Windeln sind bevorzugt Einwegwindeln. Andere Hygieneartikel mit einem absorbierenden Kern sind beispielsweise Damenbinden, Pants und Hygienevorlagen.

Ein absorbierender Kern weist ein körperseitiges Deckblatt, ein körperfernes Rückenblatt und eine zwischen dem Deckblatt und dem Rückenblatt angeordnete Schicht mit Saugmaterial auf. Die Schicht mit Saugmaterial umfasst üblicher Weise einen Superabsorber. Sie kann auch Zellstoff umfassen. Der Superabsorber und ggf. der Zellstoff sind verantwortlich für die Flüssigkeitsaufnahme und dafür, dass der absorbierende Kern sich auch in einem teilweise gefüllten Zustand nicht nass anfühlt. Bei derartigen absorbierenden Kernen ist es bekannt, Kanäle vorzusehen, die eine Flüssigkeitsverteilung in Längsrichtung unterstützen.

Der bekannte absorbierende Kern (EP 3 403 626 A1), von dem die Erfindung ausgeht, weist derartige Kanäle in diversen Formen auf.

Der bekannte absorbierende Kern weist diverse Saugkanäle auf. Bei diesen besteht jedoch hinsichtlich des Flüssigkeitsmanagements und des Tragekomforts, insbesondere in einem teilweise nassen Zustand, noch Potential.

Der Erfindung liegt das Problem zu Grunde, den bekannten absorbierenden Kern hinsichtlich seines Tragekomforts und seines Flüssigkeitsmanagements zu verbessern.

Das obige Problem wird bei einem absorbierenden Kern gemäß dem Oberbegriff von Anspruch 1 durch die Merkmale des kennzeichnenden Teils von Anspruch 1 gelöst.

Wesentlich ist die grundsätzliche Überlegung, dass absorbierende Kerne häufig in einem teilweise gefüllten Zustand getragen werden. Hinzu kommt, dass das Saugmaterial in einem nassen Zustand deutlich an Volumen zunimmt, wodurch sich die Passform verändert. Durch eine gezielte Verteilung geringer Flüssigkeitsmengen kann der Tragekomfort verbessert werden.

Durch eine Kombination verschiedener Saugkanäle mit unterschiedlichen spezifischen Kapazitäten an Saugmaterial, kann die Passform des absorbierenden Kerns in einem teilweise feuchten Zustand verbessert werden. Über Bahnen zwischen den Saugkanälen und deren Eigenschaften lässt sich steuern, welche Saugkanäle zuerst gefüllt werden.

Im Einzelnen wird vorgeschlagen, dass der absorbierende Kern, insbesondere die Schicht mit Saugmaterial, in Längsrichtung Zonen mit unterschiedlicher spezifischer Kapazität aufweist und dass der absorbierende Kern in dem ersten Abschnitt und in dem zweiten Abschnitt zumindest teilweise unterschiedliche spezifische Kapazitäten aufweist.

Bevorzugte Ausgestaltungen sind Gegenstand der Unteransprüche. Diese enthalten einige Aspekte, die vorliegend zwar als Unteransprüche aufgeführt, grundsätzlich jedoch auch unabhängig von der beschriebenen Lehre beanspruchbar sind.

Besondere Bedeutung kommt der Kombination aus Faltung und Flüssigkeitsmanagement des absorbierenden Kerns zu. Gemäß Anspruch 2 kann daher vorgesehen sein, dass Übergangszonen zwischen den Zonen mit unterschiedlicher spezifischer Kapazität vorgesehen sind, die scharfe Kanten beim Falten und Flüssigkeitsansammlungen vermeiden können. Anspruch 3 konkretisiert bevorzugte Positionen der Übergangszonen.

Die Ansprüche 4 bis 7 geben weiter bevorzugte Mengen an Saugkanälen in unterschiedlichen Abschnitten des absorbierenden Kerns an. Diese haben sich als guter Mittelweg zwischen Flüssigkeitsmanagement und Faltung ergeben. Anspruch 5 betrifft dabei einen bevorzugten Reserve-Saugkanal. Dieser kann in einem Bereich des absorbierenden Kerns angeordnet sein, in dem eine Volumenausdehnung durch Flüssigkeitsaufnahme besonders störend ist.

Eine bevorzugte Ausgestaltung, wonach die drei Saugkanäle des ersten Abschnitts jeweils in einer Übergangszone zwischen einem Bereich mit größerer spezifischer Kapazität in Längsrichtung in Richtung der Mitte des absorbierenden Kerns und einem Bereich mit geringerer spezifischer Kapazität angeordnet sind, und vorzugsweise in dem Bereich mit geringerer spezifischer Kapazität angeordnet sind, vorzugsweise, wobei die zwei Saugkanäle des zweiten Abschnitts in dem Bereich mit größerer spezifischer Kapazität angeordnet sind, weiter vorzugsweise, dass die Bereiche mit einem Saugkanal in den Bereichen mit geringerer spezifischer Kapazität angeordnet sind, betrifft den Saugkanälen zugeordnete spezifische Kapazitäten des absorbierenden Kerns. Insbesondere ist dabei gemäß Anspruch 9 mittig eine größere spezifische Kapazität vorgesehen. Im Bereich mittig zwischen den Beinen stört eine Volumenausdehnung häufig weniger. Dazu passende Mengen an Saugkanälen, auch im Hinblick auf die Faltung, gibt Anspruch 8 an.

Bevorzugte Ausgestaltungen der spezifischen Kapazität bezogen auf die Breite des absorbierenden Kerns gibt Anspruch 10 an. Eine Kombination aus zwei und drei Saugkanälen hat sich als besonders vorteilhaft erwiesen. Bevorzugt ist es daher so, dass der absorbierende Kern in dem Bereich mit geringerer Breite, insbesondere in dem gesamten Bereich, genau zwei Saugkanäle aufweist, und/oder, dass in dem Bereich mit größerer Breite der absorbierende Kern abschnittsweise genau einen, und vorzugsweise abschnittsweise genau drei, Saugkanal oder Saugkanäle aufweist.

Die Ansprüche 11 bis 13 geben bevorzugte Ausgestaltungen der Bahnen an. Diese können insbesondere anatomisch angepasst sein und/oder durch Kanäle gebildet werden.

Nach einer weiteren Lehre gemäß Anspruch 14, der eigenständige Bedeutung zukommt, wird ein Hygieneartikel mit einem vorschlagsgemäßen absorbierenden Kern beansprucht. Auf alle Ausführungen zu dem vorschlagsgemäßen absorbierenden Kern darf verwiesen werden.

Nach einer weiteren Lehre gemäß Anspruch 15, der ebenfalls eigenständige Bedeutung zukommt, wird ein Herstellungsverfahren für einen vorschlagsgemäßen absorbierenden Kern beansprucht. Auf alle Ausführungen zu dem vorschlagsgemäßen absorbierenden Kern darf verwiesen werden.

Wesentlich bei dem Herstellungsverfahren ist insbesondere die Herstellung der Bahnen. Im Einzelnen wird dabei vorgeschlagen, dass das körperseitige Deckblatt oder das körperferne Rückenblatt in eine mit einer Unterdruckquelle verbundene Form eingelegt wird, dass die Form mindestens einen Volumeneinsatz in Form der Bahnen aufweist, dass auf das Deckblatt oder das Rückenblatt Saugmaterial aufgebracht wird, dass im Bereich des Volumeneinsatzes weniger oder kein Saugmaterial aufgebracht wird oder das Saugmaterial im Bereich des Volumeneinsatzes von der Unterdruckquelle nicht oder weniger angesogen wird und so nicht oder weniger im Bereich des Volumeneinsatzes haften bleibt, vorzugsweise, dass die Form mittels einer Bürste abgebürstet wird, wodurch die Tiefe des Saugmaterials homogenisiert und der Volumeneinsatz, zumindest großteils, insbesondere vollständig, von Saugmaterial befreit wird, dass das Deckblatt, ggf. das Saugmaterial und das Rückenblatt miteinander verbunden, insbesondere verklebt werden.

Ähnliche Herstellungsverfahren sind beispielsweise aus der EP 3 342 386 A1 und der US 2015/0223991 A1 bekannt. Zur Erläuterung des Herstellungsverfahrens wird auf diese Schriften verwiesen. Beide Schriften werden vorliegend durch Referenz einbezogen. Daraus nicht bekannt ist die Nutzung der Bürste.

Im Folgenden wird die Erfindung anhand einer lediglich Ausführungsbeispiele darstellenden Zeichnung näher erläutert. In der Zeichnung zeigt
- Fig. 1: eine Windel mit einem vorschlagsgemäßen absorbierenden Kern,
- Fig. 2: a) eine Windel mit vorschlagsgemäßem absorbierenden Kern im angelegten Zustand, b) im gefalteten Zustand ähnlich dem angelegten Zustand und c) dazu korrespondierend eine Windel-Hose,
- Fig. 3: den Schichtaufbau einer Windel,
- Fig. 4: einen absorbierenden Kern in Draufsicht und Seitenansicht,
- Fig. 5: mehrere absorbierende Kerne in Draufsicht mit unterschiedlichen Faltungsstrukturen und Kanalsystemen,
- Fig. 6: weitere Faltungsstrukturen,
- Fig. 7: den Faltungsstrukturen zugeordnete Prägelinien,
- Fig. 8: die Faltungswirkung der Faltungsstrukturen,
- Fig. 9: mögliche Verklebungen des absorbierenden Kerns, insbesondere zwischen dem Deckblatt und der Schicht mit Saugmaterial,
- Fig. 10: mögliche Verklebungen des absorbierenden Kerns, insbesondere zwischen dem Rückenblatt und der Schicht mit Saugmaterial,
- Fig. 11: diverse Verjüngungen der Kanäle und/oder Faltungsbahnen und
- Fig. 12: Kanäle mit Vergrößerung der Seitenfläche.

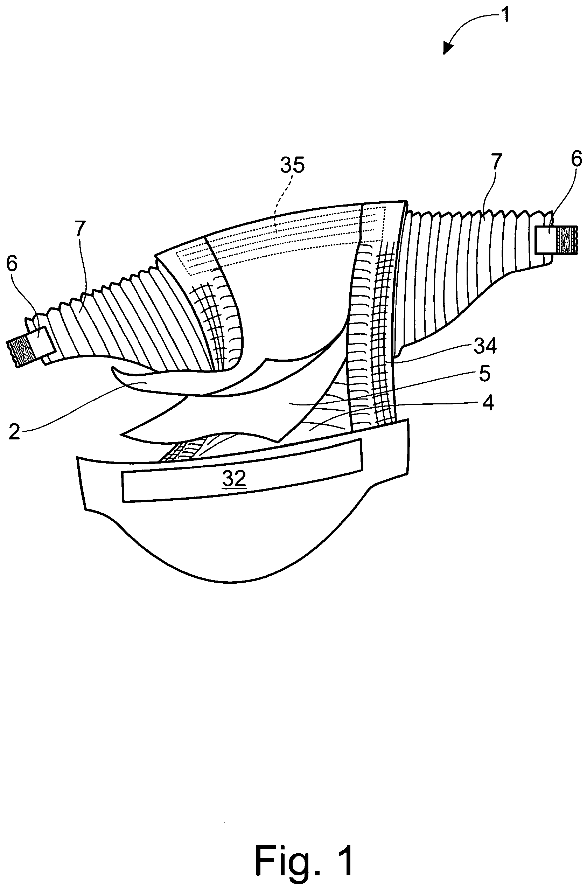

In der perspektivischen Ansicht gemäß Fig. 1 ist beispielhaft eine Windel als Hygieneartikel 1 dargestellt. Bezüglich weiterer bevorzugter Hygieneartikel 1 wird auf den einleitenden Teil der Beschreibung verwiesen. Besonders bevorzugt ist der Hygieneartikel 1 eine Baby- oder Kleinkinder-Windel. Einige bevorzugte Ausführungsbeispiele sind bezüglich der relativen und absoluten Abmessungen speziell auf die Anatomie von Babys und Kleinkindern abgestimmt.

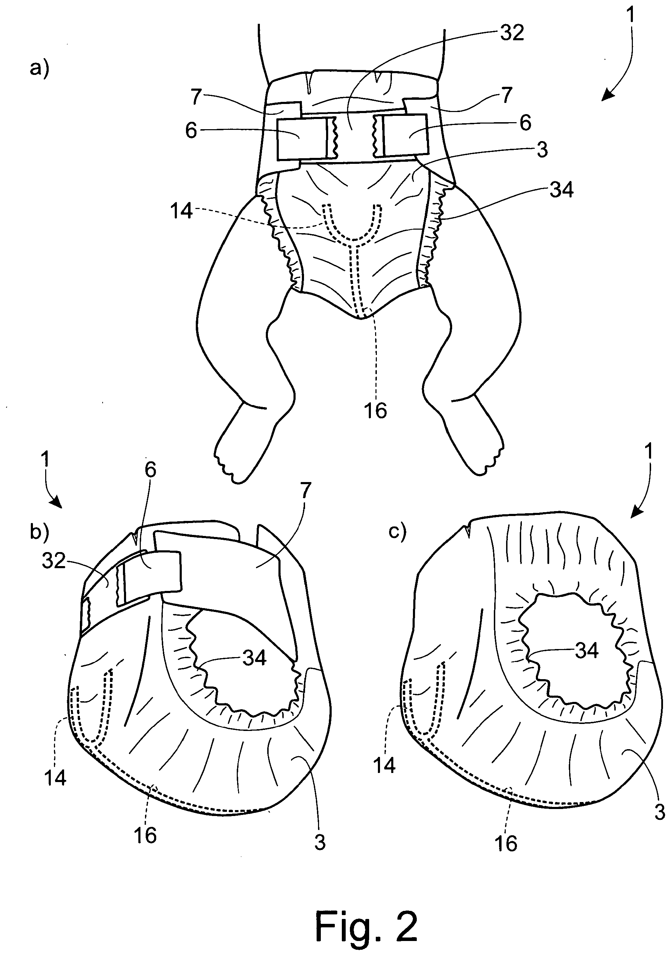

Grundsätzlich kann der Hygieneartikel 1, insbesondere die Windel, ein flüssigkeitsdurchlässiges Topsheet 2 und ein flüssigkeitsundurchlässiges Backsheet 3 aufweisen. Das Topsheet 2 ist dabei im bestimmungsgemäßen Zustand des Hygieneartikels 1 körperseitig angeordnet und lässt entsprechend Körperflüssigkeiten durch, die der Hygieneartikel 1 aufnehmen soll. Das Backsheet 3 ist beispielsweise in Fig. 2 sichtbar.

Der Hygieneartikel 1 nimmt den im Folgenden im Fokus stehenden absorbierenden Kern 4 auf. Dieser wiederum dient dazu, Körperflüssigkeiten aufzunehmen.

Der Hygieneartikel 1 nimmt den absorbierenden Kern 4 zwischen dem Topsheet 2 und dem Backsheet 4 auf. Weiterhin kann zwischen dem Topsheet 2 und dem absorbierenden Kern 4 eine Verteilerlage 5 angeordnet sein.

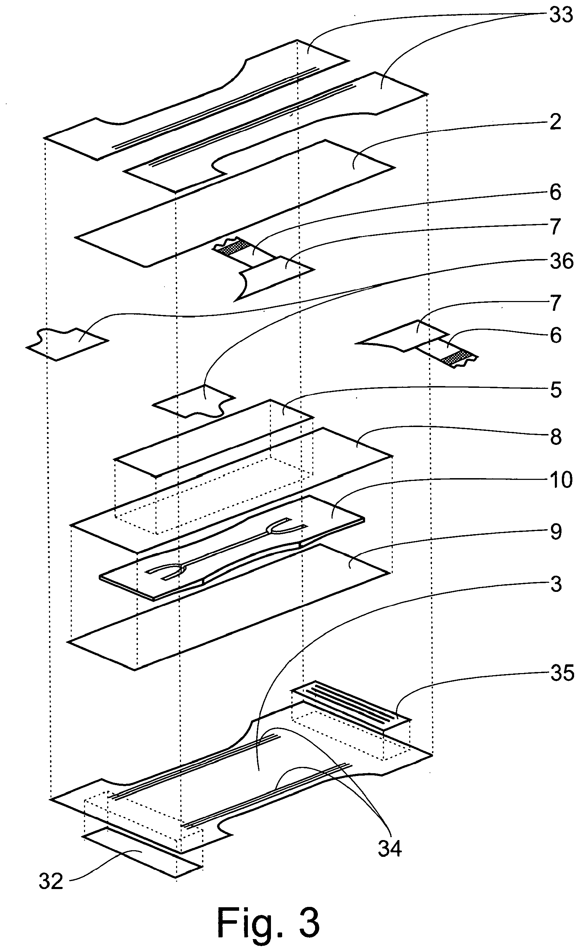

Der Hygieneartikel 1 kann Befestigungslaschen 6 aufweisen, die an Seitenflügeln 7 des Hygieneartikels 1 angeordnet sind. Die Seitenflügel 7 können mit dem Hygieneartikel 1, insbesondere dem Backsheet 3, verbunden, insbesondere verklebt, oder einstückig damit ausgeführt, sein. Die Befestigungslaschen 6 können, wie in Fig. 2 a) gezeigt, zum gürtelartigen Verschließen des Hygieneartikels 1, insbesondere der Windel, im Hüftbereich dienen. Wie Fig. 2 c) zeigt, sind jedoch durchaus auch andere Ausführungen für Hygieneartikel 1 und Windeln bekannt. Weitere Details des insbesondere als Windel ausgestalteten Hygieneartikels 1 wie der Schichtaufbau gemäß Fig. 3 werden im Folgenden noch erläutert.

Im Fokus steht nun der absorbierende Kern 4. Der absorbierende Kern 4 bildet das Herzstück des Hygieneartikels 1 und trägt wesentlich zu dessen Funktion und Passform bei.

Der absorbierende Kern 4 weist ein körperseitiges Deckblatt 8, ein körperfernes Rückenblatt 9 und eine zwischen dem Deckblatt 8 und dem Rückenblatt 9 angeordnete Schicht 10 mit Saugmaterial auf. Im Folgenden werden zuerst diverse Ausführungsformen einer ersten Lehre erläutert, die mit der vorschlagsgemäßen Lehre kompatibel sind und diese teilweise aus einem anderen Blickwinkel betrachten. Diese Aspekte weisen für sich eigene Relevanz auf, die hier nicht beansprucht, aber beanspruchbar, ist. Anschließend wird die vorschlagsgemäße Lehre erläutert.

Das Deckblatt 8 und/oder das Rückenblatt 9 können Materialen aufweisen gewählt aus der Liste Polypropylen (PP), Polyethylenterephthalat (PET), Polyethylen (PET), Naturfasern, Chemiefasern aus natürlichen und/oder synthetischen Filamenten, Vliese, insbesondere Spinnvliese und kardierte Vliese, Lochfolien, Kombinationen aus Folien, insbesondere Lochfolien, und Vlies. Die Vliese können jeweils thermisch verbunden, wasserstrahlverfestigt, mit Harz verbunden, als Nadelvlies ausgestaltet, mit Ultraschall verbunden oder durch Kombinationen daraus hergestellt sein. Hier und vorzugsweise weist das Deckblatt 8 und/oder das Rückenblatt 9 ein Flächengewicht von 6 gsm bis 25 gsm auf.

Das Saugmaterial umfasst hier und vorzugsweise einen Superabsorber. Zusätzlich kann das Saugmaterial Zellstoff umfassen. Hier und vorzugsweise umfasst das Saugmaterial mindestens 50 Gew.-%, vorzugsweise mindestens 60 Gew.-%, weiter vorzugsweise mindestens 70 Gew.-%, und/oder höchstens 99 Gew.-%, vorzugsweise höchstens 90 Gew.-%, Superabsorber und/oder höchstens 50 Gew.-%, vorzugsweise höchstens 40 Gew.-%, weiter vorzugsweise höchstens 30 Gew.-%, und/oder mindestens 1 Gew.-%, vorzugsweise mindestens 10 Gew.-%, Zellstoff.

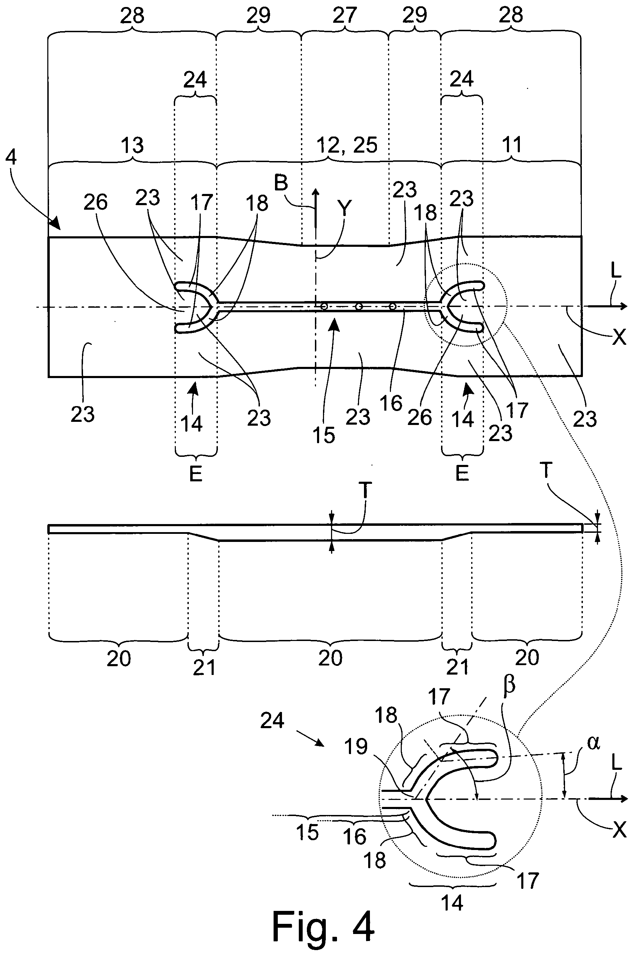

Die im Folgenden verwendeten geometrischen Bezeichnungen des absorbierenden Kerns 4 werden anhand von Fig. 4 erläutert. Der absorbierende Kern 4 weist eine Längsrichtung L mit einer longitudinalen Mittellinie X und zur Längsrichtung L orthogonale Breitenrichtung B auf. Die Längsrichtung L und die Breitenrichtung B spannen die Fläche des absorbierenden Kerns 4 in der Draufsicht auf und sind im angelegten Zustand etwa orthogonal zur Körperfläche des Trägers. Die Längsrichtung L entspricht dabei im angelegten Zustand der Richtung von vorne nach hinten beziehungsweise hinten nach vorne, während die Breitenrichtung B von Bein zu Bein zeigt. Hier und vorzugsweise ist der absorbierende Kern 4 in Längsrichtung L länger als in Breitenrichtung B.

Der absorbierende Kern 4 kann einen vorderen Abschnitt 11, einen mittleren Abschnitt 12 und einen hinteren Anschnitt 13 entlang der Längsrichtung L aufweisen. Der mittlere Abschnitt 12 ist auch als crotch-Bereich bekannt. Wie sich im Folgenden noch ergeben wird, sind die Abschnitte 11, 12, 13 funktional definiert und können entsprechend je nach absorbierendem Kern 4 unterschiedliche Längen in Längsrichtung L aufweisen. Es ist auch so, dass in manchen Ausführungsformen die Grenze zwischen mittlerem Abschnitt 12 und vorderem Abschnitt 11 oder hinterem Abschnitt 13 funktional offengelassen ist. In diesen Fällen kommt es darauf jedoch auch nicht an.

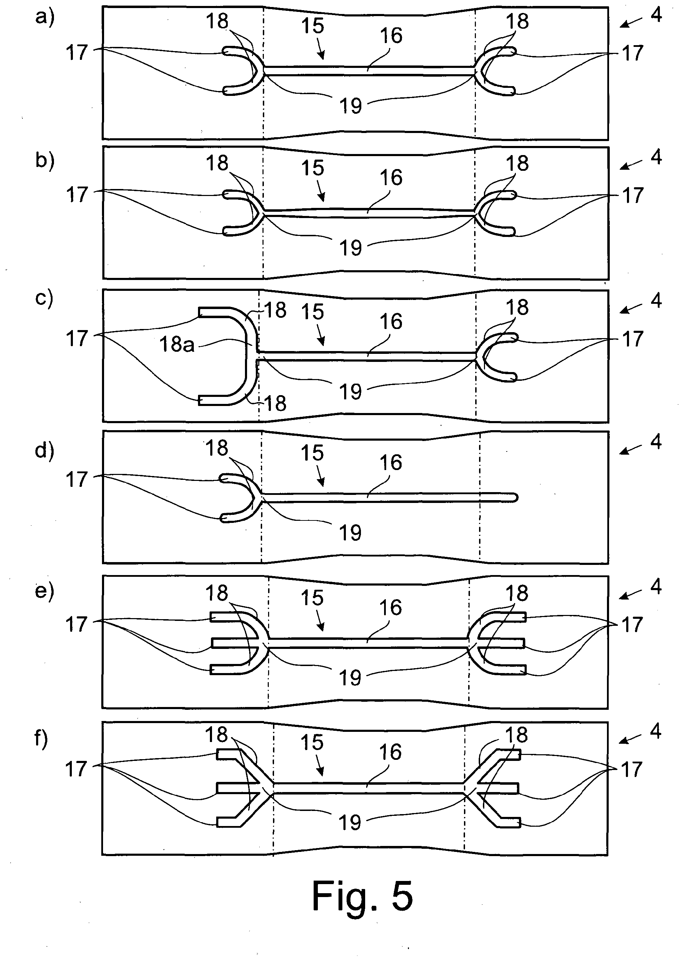

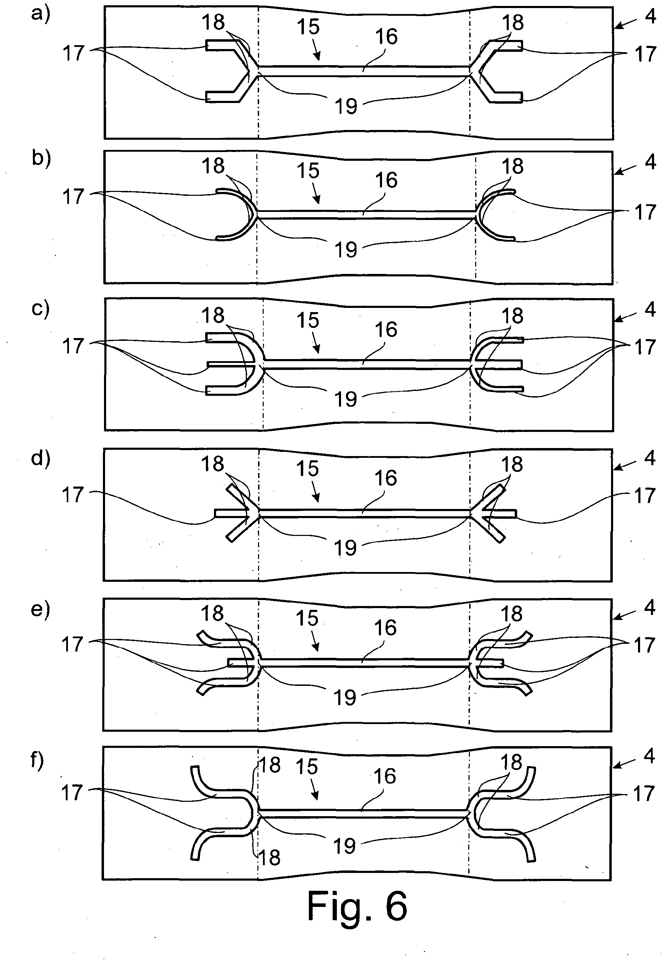

Gemäß der ersten Lehre weist der absorbierende Kern 4 im vorderen und/oder im hinteren Abschnitt 11, 13 mindestens eine Faltungsstruktur 14 auf. Hier und vorzugsweise weist der absorbierende Kern 4 im vorderen und im hinteren Abschnitt 11, 13 jeweils eine Faltungsstruktur 14 auf. Beispielhafte Faltungsstrukturen 14 finden sich insbesondere in den Figuren 5 und 6. Die Faltungsstruktur 14 oder die Faltungsstrukturen 14 kann beziehungsweise können U-förmig ausgestaltet sein. So oder so unterstützt die Faltungsstruktur 14, gegebenenfalls jeweils, eine körperadaptierte Faltung des absorbierenden Kerns 14 und somit insbesondere des Hygieneartikels 1. Diverse Ausgestaltungen und Kombinationen der Faltungsstruktur 14 werden im Folgenden noch erläutert.

Die Faltungsstruktur 14 weist eine Längserstreckung E auf, die den vorderen und/oder hinteren Abschnitt 11, 13 des absorbierenden Kerns 4, insbesondere jeweils, definiert. Sofern nur vorne oder nur hinten eine Faltungsstruktur 14 vorgesehen ist, bleibt die Definition des jeweils anderen Abschnitts 11, 13 gegebenenfalls offen. Wird dieser Abschnitt 11, 13 nicht durch andere Merkmale definiert, kommt es darauf jedoch auch nicht an. Die Abschnitte 11, 12, 13 sind insofern nur notwendig, um die relative Lage diverser Merkmale zueinander zu beschreiben. Wie in Fig. 4 beispielsweise ersichtlich ist, definiert die Längserstreckung E der Faltungsstruktur 14 jeweils die Grenze des vorderen beziehungsweise hinteren Abschnitts 11, 13 zum mittleren Abschnitt 12 nicht aber dessen Ende nach außen in Längsrichtung L.

Der mittlere Abschnitt 12 schließt sich an den vorderen Abschnitt 11 und an den hinteren Abschnitt 13 an. Der mittlere Abschnitt 12 umfasst zumindest eine in Breitenrichtung B verlaufende transversale Mittellinie Y des absorbierenden Kerns 4. Im Umkehrschluss liegen daher der vordere und der hintere Abschnitt 11, 13 jeweils beidseits der transversalen Mittellinie Y des absorbierenden Kerns 4. Daraus folgt auch, dass die Faltungsstruktur 14 sich entlang der Längsrichtung L nicht durch die in Breitenrichtung B verlaufende Mittellinie Y des absorbierenden Kerns 4 erstreckt.

Die longitudinale Mittellinie X und die transversale Mittellinie Y teilen den absorbierenden Kern 4 jeweils in zwei gleichgroße Teilabschnitte in Breitenrichtung B bzw. Längsrichtung L. Beide sind orthogonal zueinander.

Weiterhin ist bei der ersten Lehre vorgesehen, dass der absorbierende Kern 4 ein Kanalsystem 15 mit mindestens einem Kanal 16 aufweist. Das Kanalsystem 15 dient zur Flüssigkeitsverteilung entlang der Längsrichtung L. Hier und vorzugsweise weist das Kanalsystem 15 genau einen Kanal 16 auf.

Der mindestens eine Kanal 16 erstreckt sich durch mindestens einen die transversale Mittellinie Y umfassenden Teil des mittleren Abschnitts 12. Der Kanal 16 kann dabei verbunden oder abschnittsweise getrennt ausgestaltet sein. Dabei ist insbesondere auch denkbar, dass auf Höhe der transversalen Mittellinie Y gerade ein Abschnitt des Kanals 16 liegt, in dem kein Kanal vorhanden ist. Der Kanal 16 weist jedoch entlang mindestens 50% seiner Länge tatsächlich Kanalstücke auf. Vorzugsweise weist er entlang mindestens 75%, weiter vorzugsweise mindestens 90% seiner Länge Kanalstücke auf.

Insbesondere können auch zwei, vorzugsweise parallele, Kanäle 16 vorgesehen sein, die sozusagen einen platten Schiffsrumpf bilden. Diese sind vorzugsweise beide innerhalb der mittleren 30% der Breite des absorbierenden Kerns 4 angeordnet.

Alle Ausführungen zur Breite des absorbierenden Kerns 4 können sich auch alternativ auf, ggf. nur, die Breite der Schicht 10 mit Saugmaterial beziehen. Es kann also insbesondere vorgesehen sein, dass das Deckblatt 8 und das Rückenblatt 9 rechteckig sind und aus funktionalen Gründen nur die Schicht 10 mit Saugmaterial wie in den Figuren gezeigt und noch erläutert wird variierende Breiten aufweist.

Vorzugsweise ist es nun so, dass die Faltungsstruktur 14 im vorderen und/oder hinteren Abschnitt 11, 13 mindestens zwei longitudinale Faltungsbahnen 17 und zwei schiefe Faltungsbahnen 18 aufweist. Vorzugsweise sind diese alle miteinander verbunden. Hier und vorzugsweise ist je eine longitudinale Faltungsbahn 17 und je eine schiefe Faltungsbahn 18 beidseits der longitudinalen Mittellinie X angeordnet.

Die schiefen Faltungsbahnen 18 im vorderen und/oder hinteren Abschnitt 11, 13 sind in Breitenrichtung B zwischen den longitudinalen Faltungsbahnen 17 angeordnet. Das schließt nicht aus, dass sich weitere schiefe Faltungsbahnen ausgehend von den longitudinalen Faltungsbahnen 17 nach außen erstrecken. Diese sind dann jedoch nicht mehr Teil der beschriebenen schiefen Faltungsbahnen 18. Die schiefen Faltungsbahnen 18 erstrecken sich jeweils in die mittleren 10% der Breite des absorbierenden Kerns 4. Wie noch beschrieben wird, treffen sie sich insbesondere in der Mitte des absorbierenden Kerns 4.

Weiterhin möglich ist, dass die longitudinalen Faltungsbahnen 17 im vorderen und/oder hinteren Abschnitt 11, 13 sich in Längsrichtung L ausgehend von den schiefen Faltungsbahnen 18 von der Mitte des absorbierenden Kerns 4 weg erstrecken. Auch dabei gilt, dass weitere longitudinale Faltungsbahnen sich in Richtung der Mitte des absorbierenden Kerns 4 an die schiefen Faltungsbahnen 18 anschließen können, diese dann jedoch nicht Teil der beschriebenen longitudinalen Faltungsbahnen 17 sind. Es ist damit so, dass sich die Längserstreckung E der Faltungsstruktur 14 auf der Seite der Mitte des absorbierenden Kerns 4 und somit die Definition des vorderen beziehungsweise hinteren Abschnitts 11, 13 durch die schiefen Faltungsbahnen 18 und deren Erstreckung in Richtung der Mitte des absorbierenden Kerns 4 ergibt.

Es kann auch vorgesehen sein, dass die longitudinalen Faltungsbahnen 17 im vorderen und/oder hinteren Abschnitt 11, 13 jeweils zumindest abschnittsweise einen Winkel α von höchstens 30° gegenüber der longitudinalen Mittellinie X aufweisen und die schiefen Faltungsbahnen 18 im vorderen und/oder hinteren Abschnitt 11, 13 jeweils, zumindest abschnittsweise einen Winkel β von 30° bis 60° gegenüber der longitudinalen Mittellinie X aufweisen. Der Winkel α der Faltungsbahnen 17, 18 gegenüber der longitudinalen Mittellinie X ist dabei so definiert, dass eine Linie parallel zur longitudinalen Mittellinie X einen Winkel von 0° aufweist und orthogonal dazu einen Winkel von 90°. Hier und vorzugsweise verlaufen die longitudinalen Faltungsbahnen 17 und/oder die schiefen Faltungsbahnen 18 von der longitudinalen Mittellinie aus nach außen. Der Einfachheit halber sind die Winkel nur in Fig. **4** **einmal dargestellt. Der Winkel α der longitudinalen Faltungsbahn 17 beträgt dabei etwa 0° und der Winkel β der** schiefen Faltungsbahnen 18 etwa 45 °.

Diese erste Lehre lässt sich am Beispiel einer Windel gut erläutern. Im mittleren Abschnitt 12 des absorbierenden Kerns 4, der dem Bereich zwischen den Beinen des Trägers entspricht, sollte die Windel eine andere Passform bilden, als im vorderen und/oder hinteren Abschnitt 11, 13. Daher wird die Faltungsstruktur 14 so ausgestaltet, dass sich im vorderen Abschnitt 11, also im unteren Bauchbereich, und/oder im hinteren Abschnitt 13 eine gewölbte, insbesondere trapezförmige, Passform ergibt. Dabei bildet die Faltungsstruktur 14 Faltungsbahnen 17, 18 aus, an denen der absorbierende Kern 4 beziehungsweise die Windel knickt. Dies allein ist jedoch nicht ausreichend. Weiterhin soll die Faltungsstruktur 14 im vorderen und/oder hinteren Abschnitt 11, 13 an das Kanalsystem 15 angepasst werden, das ebenfalls, in Form von Kanälen 16, Faltungsbahnen ausbildet. Ohne eine derartige Anpassung bilden sich unerwünschte Verwerfungen, die den Tragekomfort vermindern. Die Anpassung wird durch schiefe Faltungsbahnen 18 erreicht.

Entsprechend ergibt sich daher zwischen einem gewölbten Bereich, der durch die longitudinalen Faltungsbahnen 17 gebildet wird, ein Übergangsbereich, der durch die schiefen Faltungsbahnen 18 gebildet wird und der einen komfortablen Übergang zwischen dem vorderen und/oder hinteren Abschnitt 11, 13 und dem mittleren Abschnitt 12 des absorbierenden Kerns 4 in geknickter Form bietet. Insbesondere kann der Hygieneartikel 1, vorzugsweise eine Windel, somit im gefalteten, angelegten Zustand eine Schiffsform einnehmen. Mittig zwischen den Beinen des Trägers ergibt sich ein Schiffsrumpf, der in Breitenrichtung B vorzugsweise dreieckig ist, oder ein invertierter Schiffsrumpf, also eine korrespondierende Form, die jedoch nach innen statt nach außen geknickt ist. Vorne und/oder hinten hingegen ergibt sich eine eher flächige, ebenfalls an die menschliche Anatomie angepasste, Form. Diese beiden Formen gehen graduell ineinander über, wodurch sich eine unerwünschte Faltenbildung vermeiden lässt.

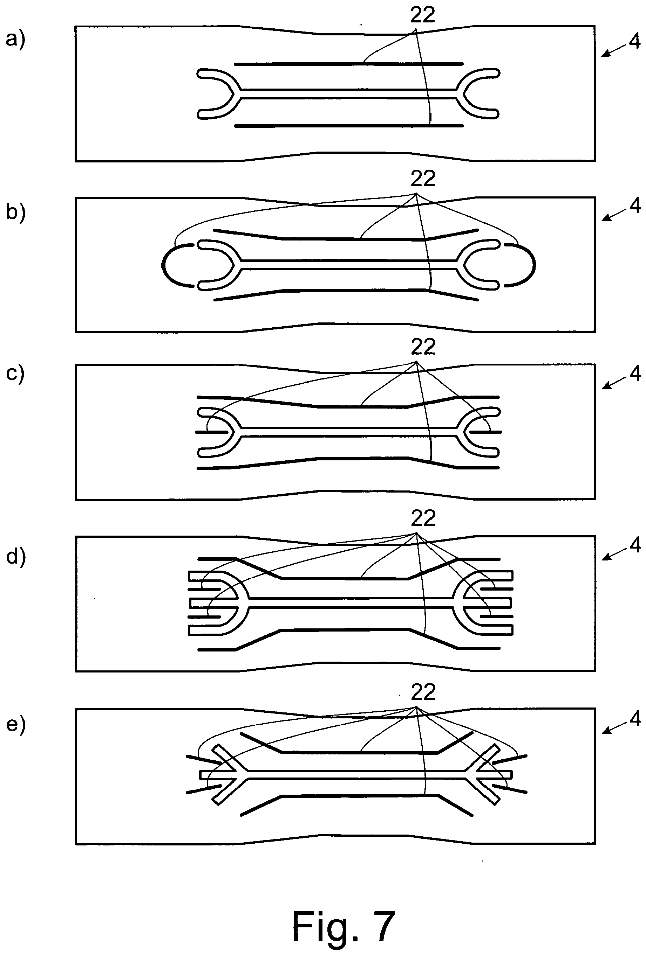

Es wird darauf hingewiesen, dass hier auch diverse Effekte und Merkmale in Kombination mit der ersten Lehre beschrieben werden, die für sich auch eigenständige Relevanz aufweisen und mit der vorschlagsgemäßen und der ersten Lehre kombiniert werden können, jedoch nicht müssen. Ausdruck findet dies zum Beispiel in der Fig. 6 d) und der Fig. 7 e), die jeweils nur eine longitudinale Faltungsbahn 17 aufweisen, die außerdem nicht zu den longitudinalen Faltungsbahnen 17 der ersten Lehre korrespondiert.

Die Faltungseffekte der ersten Lehre werden im Folgenden anhand der Ausführungsbeispiele noch näher erläutert. Diese sind auch mit der vorschlagsgemäßen Lehre erzielbar.

Die Figuren 5 und 6 zeigen diverse Ausgestaltungen von Faltungsstrukturen 14. Wiederkehrend ist dabei die bevorzugte U-Form. Auch gezeigt ist, dass die Faltungsstruktur 14 nur einseitig angeordnet sein kann. Ebenso bevorzugt, ist, dass die Faltungsstrukturen 14 vorn und hinten voneinander unterschiedlich sein können. Dabei kann auch vorgesehen sein, dass nur eine der Faltungsstrukturen 14 nach der beschriebenen ersten Lehre ausgestaltet ist. Dies kann dem Umstand Rechnung tragen, dass die menschliche Anatomie vorne und hinten unterschiedlich ist.

In Anbetracht der Größenverhältnisse eines üblichen absorbierenden Kerns 4 im Verhältnis zur Anatomie und unter Berücksichtigung des Umstandes, dass der mittlere Abschnitt 12 zumeist länger als der vordere und der hintere Abschnitt 11, 13 sein wird, ist vorzugsweise vorgesehen, dass die Längserstreckung E der Faltungsstruktur 14 höchstens 80%, vorzugsweise höchstens 50%, weiter vorzugsweise höchstens 30%, der Länge des mittleren Abschnitts 12 und/oder des Kanals 16 beträgt.

In diversen bevorzugten Ausführungsbeispielen sind die schiefen Faltungsbahnen 18 konvex geschwungen. So wird ein sanftes Auslaufen der Faltung in den äußeren Bereichen des absorbierenden Kerns 4 ermöglicht. Weiterhin entspricht die Konvexität der menschlichen Anatomie.

Vorzugsweise werden die Faltungsbahnen 17, 18 im vorderen und/oder hinteren Abschnitt 11, 13 jeweils durch einen Kanal gebildet. Es kann also vorgesehen sein, dass alle in den Figuren 5 und 6 gezeigten Faltungsbahnen 17, 18 Kanäle sind. Bevorzugt ist dabei, dass die Kanäle im vorderen und/oder hinteren Abschnitt 11, 13 jeweils miteinander und dem mindestens einen Kanal 16 im mittleren Abschnitt 12 flüssigkeitsleitend kommunizieren, insbesondere verbunden sind. So kann ein Flüssigkeitstransfer zwischen dem vorderen Abschnitt 11 und/oder dem hinteren Abschnitt 13 und dem mittleren Abschnitt 12 erreicht werden. Dieser Flüssigkeitstransfer kann zum einen so ausgestaltet sein, dass sich Flüssigkeit vor allem im mittleren Abschnitt 12 sammelt und andererseits so, dass bei Eintritt größerer Flüssigkeitsmengen diese in den vorderen und/oder hinteren Abschnitt 11, 13 ausweichen können.

Wie noch erläutert wird, weist der mittlere Abschnitt 12 vorzugsweise mehr Saugmaterial, insbesondere mehr Superabsorber, auf. Aus Gründen des Tragekomforts und insbesondere des Liegekomforts kann es beabsichtigt sein, im vorderen und/oder hinteren Abschnitt 11, 13 weniger Flüssigkeit oder erst zu einem späteren Zeitpunkt Flüssigkeit zu sammeln. Um ein Laufen der Flüssigkeit gerade im Liegen in den vorderen und/oder hinteren Abschnitt 11, 13 zu verhindern, kann eine noch zu erläuternde Ventilfunktion vorgesehen sein. Es wird darauf hingewiesen, dass dieser Ventilfunktion in allen Ausführungsformen eigene Relevanz aufweist.

Insbesondere bezüglich der Faltung sind hier und vorzugsweise genau zwei oder genau drei longitudinale Faltungsbahnen 17 vorgesehen.

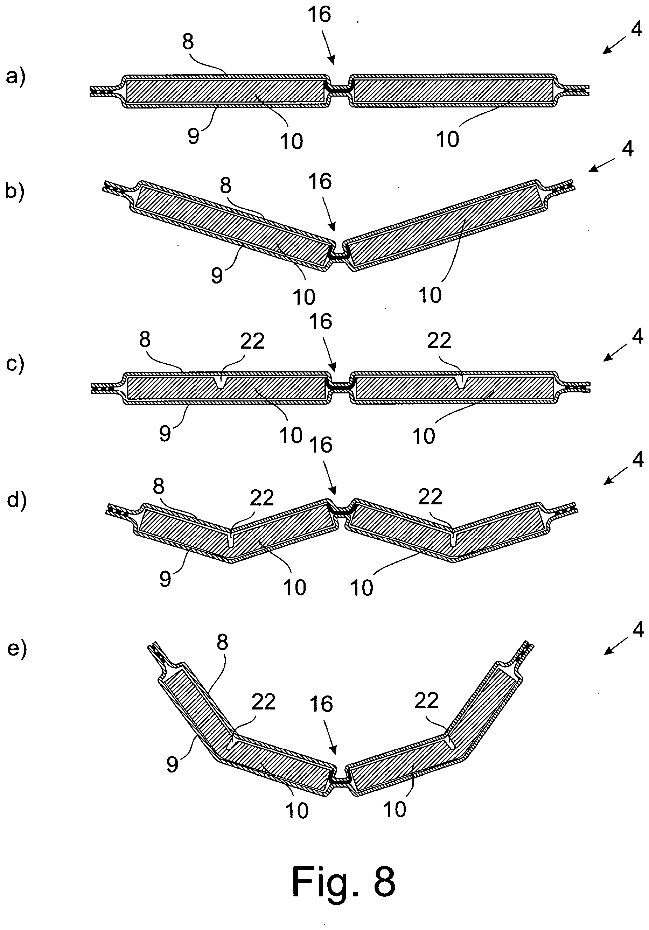

Ein Kanal ist hier definiert als ein Bereich, der eine höhere Fließgeschwindigkeit und/oder eine höhere Fließkapazität für Flüssigkeiten in Längsrichtung aufweist. Vorzugsweise ist der Kanal im Wesentlichen oder vollständig frei von Superabsorber und/oder dem Saugmaterial. Hier und vorzugsweise sind im Kanalbereich das Deckblatt 8 und das Rückenblatt 9 fest miteinander verbunden. Vorzugsweise lösen sich das Deckblatt 8 und das Rückenblatt 9 im normalen Gebrauch im Bereich der Kanäle nicht voneinander. Das Deckblatt 8 und das Rückenblatt 9 können miteinander verklebt oder auch thermisch oder durch Ultraschallschweißen verbunden sein. Im Querschnitt sind die Kanäle in Fig. 8 zu sehen.

Im Folgenden werden Ausgestaltungen der longitudinalen Faltungsbahnen 17 näher erläutert.

Es kann vorgesehen sein, dass die longitudinalen Faltungsbahnen 17 im vorderen und/oder hinteren Abschnitt 11, 13 jeweils zumindest über 50% ihrer Längserstreckung, vorzugsweise über 80% ihrer Längserstreckung, einen Winkel α von höchstens 20°, vorzugsweise höchstens 10°, weiter vorzugsweise höchstens 5°, gegenüber der longitudinalen Mittellinie X aufweisen.

Zusätzlich oder alternativ kann vorgesehen sein, dass die longitudinalen Faltungsbahnen 17 im vorderen und/oder hinteren Abschnitt 11, 13 konvex mit einem abnehmenden Winkel α ausgestaltet sind. Sie können dabei insbesondere wie in den Figuren gezeigt parallel zur longitudinalen Mittellinie X auslaufen.

Wie bereits erwähnt, können die longitudinalen Faltungsbahnen 17 und/oder die schiefen Faltungsbahnen 18 im vorderen und/oder hinteren Abschnitt 11, 13 beidseits der longitudinalen Mittellinie X angeordnet sein und vorzugsweise in Breitenrichtung B und Längsrichtung L von der Mitte des absorbierenden Kerns 4 nach außen verlaufen.

Es kann vorgesehen sein, dass die longitudinalen Faltungsbahnen 17 im vorderen und/oder hinteren Abschnitt 11, 13 vollständig innerhalb der mittleren 70% der Breite des absorbierenden Kerns 4, vorzugsweise innerhalb der mittleren 50% der Breite des absorbierenden Kerns 4, weiter vorzugsweise innerhalb der mittleren 40% der Breite des absorbierenden Kerns 4 angeordnet sind.

Im Folgenden wird ein Aspekt der bereits erwähnten Ventilfunktion näher erläutert. Diese Ventilfunktion sorgt zum einen dafür, dass Flüssigkeit zwischen den verschiedenen Abschnitten 11, 12, 13 unter manchen Umständen langsamer ausgetauscht wird und zum anderen dafür, dass sie dennoch unter manchen Umständen schneller ausgetauscht wird, als wenn keine Kanäle vorhanden wären. Sie kann insbesondere im Liegen des Trägers durch eine scharfe Faltkante noch unterstützt werden und so ein Auslaufen oder eine Ansammlung von Flüssigkeit im vorderen und/oder hinteren Abschnitt 11, 13 verhindern oder reduzieren.

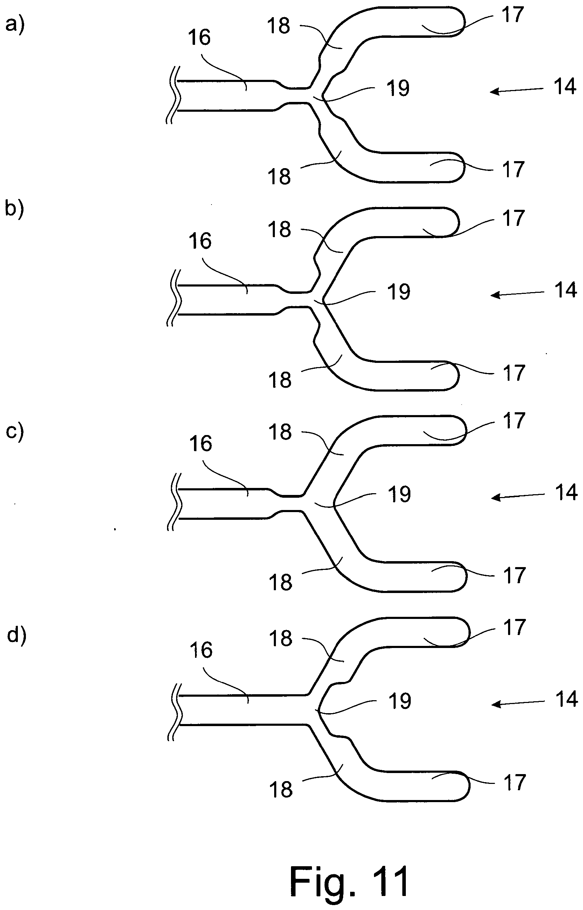

Insbesondere zur Erzielung dieser Ventilfunktion kann es vorgesehen sein, dass eine oder mehrere Faltungsbahnen 17, 18 und/oder Kanäle 16 eine sich verjüngende Breite in einem Bereich aufweisen, in dem die jeweilige Faltungsbahn 17, 18 beziehungsweise der jeweilige Kanal 16 mit einer weiteren Faltungsbahn 17, 18 und/oder einem weiteren Kanal 16 verbunden ist. Diesbezüglich wird auf Fig. 11 verwiesen. Dort ist eine Faltungsstruktur 14 mit unterschiedlichen Verjüngungen im Bereich der Faltungsbahnen 17, 18 und/oder des Kanals 16 dargestellt. Diese Verjüngung kann neben der Ventilfunktion, die sich durch den weniger breiten Kanal ergibt, dazu führen, dass gerade im Liegen der absorbierende Kern 4 und insbesondere die Windel eine scharfe Knickkante ausbildet, die Flüssigkeit großteils stoppt. Vorzugsweise ist diese Knickkante bei stehender Nutzung des absorbierenden Kerns 4, und insbesondere der Windel, weniger scharf ausgebildet oder gar nicht vorhanden. So können also unterschiedliche Faltungseigenschaften mit einer Faltungsstruktur 14 und/oder gegebenenfalls deren Anbindung an den Kanal 16 erreicht werden. Natürlich ist auch denkbar, die Verjüngungen an anderen Stellen anzuordnen, um dort Ventilfunktionen zu erzielen. Verstärkt wird die Ventilfunktion dadurch, dass das Saugmaterial im nassen Zustand die verjüngten Bereiche zuquillt.

Je nach Ausgestaltung des absorbierenden Kerns 4 und des Hygieneartikels 1 ist nicht immer eine gleichmäßige Verteilung der Flüssigkeitsaufnahme gewünscht. Geringere Mengen an Flüssigkeiten sollen beispielsweise häufig eher im mittleren Abschnitt 12 aufgenommen werden, sodass im vorderen und hinteren Abschnitt 11, 13 der absorbierende Kern 4 weniger gefüllt ist und sich somit weniger ausdehnt. Es kann also vorkommen, dass die Flüssigkeitsverteilung von einem Abschnitt 11, 12, 13 in einen anderen Abschnitt 11, 12, 13 des absorbierenden Kerns 4 bei kleineren Flüssigkeitsmengen ungewünscht, bei größeren Flüssigkeitsmengen jedoch gerade notwendig ist. Durch die Verjüngung der Kanäle 16 lässt sich diese Verteilung steuern. Hinzu kommt, dass sich über die Verjüngung von Kanälen 16 und/oder Faltungsbahnen 17, 18 die Faltungswirkung verändern lässt. Im Bereich einer Verjüngung kann sich beispielsweise auch eine Faltung quer zur entsprechenden Faltungsbahn 16, 17, 18 einstellen.

Wie in Fig. 11 dargestellt, ist vorzugsweise vorgesehen, dass die Faltungsstruktur 14 im vorderen und/oder hinteren Abschnitt 11, 13 einen Knotenpunkt 19 aufweist, an dem der Kanal 16 und die schiefen Faltungsbahnen 18 miteinander verbunden sind und dass eine oder mehrere der schiefen Faltungsbahnen 18 und/oder der Kanal 16 sich zu dem Knotenpunkt 19 hin verjüngt oder innerhalb von 20 mm, vorzugsweise 10 mm Abstand zu Knotenpunkt 19 einen Bereich mit sich verjüngender oder verjüngter Breite aufweist.

Hier und vorzugsweise ist der Knotenpunkt 19 im Verhältnis zu den Abmessungen des absorbierenden Kerns 4 derart angeordnet, dass eine Verbindungslinie vom Knotenpunkt 19 zu beiden äußeren Ecken des zugehörigen Abschnitts, also des vorderen oder hinteren Abschnitts 11, 13, einen Winkel kleiner als 40°, vorzugsweise kleiner als 30°, weiter vorzugsweise kleiner als 22,5°, und/oder größer als 15°, vorzugsweise größer als 20°, gegenüber der longitudinalen Mittellinie X aufweist. Ebenfalls bevorzugt ist, dass die Verbindungslinie durch ein Ende der jeweiligen longitudinalen Faltungsbahn oder innerhalb von 10 mm, vorzugsweise 5mm, an diesem vorbei verläuft.

Im Folgenden werden bevorzugte Ausgestaltungen der schiefen Faltungsbahn 18 weiter erläutert.

Es kann vorgesehen sein, dass die schiefen Faltungsbahnen 18 im vorderen und/oder hinteren Abschnitt 11, 13 sich jeweils in die mittleren 5% der Breite des absorbierenden Kerns 4 erstrecken, vorzugsweise sich in der Mitte der Breite des absorbierenden Kerns 4 treffen und dort verbunden sind. Zusätzlich oder alternativ kann vorgesehen sein, dass die schiefen Faltungsbahnen 18 im vorderen und/oder hinteren Abschnitt 11, 13 jeweils einen transversalen Abschnitt 18a aufweisen, der einen Winkel größer 60° gegenüber der longitudinalen Mittellinie X aufweist. Vorzugsweise verläuft der transversaleAbschnitt 18a höchstens innerhalb der mittleren 10%, vorzugsweise innerhalb der mittleren 5%, und/oder mindestens innerhalb der mittleren 3%, vorzugsweise der mittleren 5%, der Breite des absorbierenden Kerns 4. Auch dieser transversale Abschnitt 18a kann ähnlich wie die Verjüngungen zur Ventilfunktion, insbesondere durch Bilden einer Knickkante im Liegen, beitragen. Es kann auch vorgesehen sein, dass ein transversaler Abschnitt 18a alleine vorgesehen ist oder in Kombination mit anderen Abschnitten, die nicht im Bereich 30° bis 60° verlaufen. Im letzten Fall ist der transversale Abschnitt 18a dann kein Teil einer schiefen Faltungsbahn 18.

Es kann vorgesehen sein, dass die schiefen Faltungsbahnen 18 im vorderen und/oder hinteren Abschnitt 11, 13 jeweils zumindest abschnittsweise einen Winkel β von 40° bis 50° gegenüber der longitudinalen Mittellinie X aufweisen. Bevorzugt ist dabei, dass die schiefen Faltungsbahnen 18 im vorderen und/oder hinteren Abschnitt 11, 13 zumindest teilweise in einem Winkel β von 45° zur longitudinalen Mittellinie X von der Mitte des absorbierenden Kerns 4 in Längsrichtung L und Breitenrichtung B nach außen verlaufen.

Besonders bevorzugt ist es so, dass die schiefen Faltungsbahnen 18 einen Abschnitt aufweisen, der sich in die mittleren 10%, vorzugsweise 5%, der Breite des absorbierenden Kerns 4 erstreckt und der innerhalb dieser 10%, vorzugsweise 5%, in dem Winkel von 30° bis 60°, vorzugsweise 40° bis 50°, weiter vorzugsweise 45°, zur longitudinalen Mittellinie X verläuft und/oder mit einem derartigen Winkel auf der zur longitudinalen Mittellinie X weisenden Seite beginnt.

Die Vorteile der Faltungsstrukturen 14 insbesondere in Kombination mit dem Kanal 16 sind anhand einer anatomischen Betrachtung evident. Die menschliche Anatomie weist im vorderen Leistenbereich die regio pubica flankiert von je einer regio inguinalis auf. In diesem Bereich ist eine trapezförmige Form des Hygieneartikels 1 anatomisch angepasst. Die longitudinalen Faltungsbahnen 17 können diese Trapezform herstellen. Anatomisch weiter unten (kaudal) folgt der Penis oder die Vagina. In diesem Bereich und weiter bis nach hinten ist eine Aufteilung in zwei Bereiche, die Beulen nach außen oder nach innen, gegebenenfalls auch beides, ermöglicht, vorteilhaft. Alternativ kann die Faltungsstruktur 14 auch in diesem Bereich angeordnet sein und so eine Trapez-Form oder invertierte Trapez-Form bilden.

Der Übergang zwischen den Formen wird über die schiefen Faltungsbahnen 18 erzielt. Dabei sollen möglichst Verwerfungen vermieden werden. Im hinteren Abschnitt 13 kann die Faltungsstruktur 14 so ausgelegt sein, dass sie mittig ein Ausbeulen nach innen und/oder ein flächiges Anliegen an die glutealen Regionen ermöglicht. Auch hier ist entsprechend ein Übergang vorteilhaft. Es ist somit ersichtlich, dass auch im vorderen Abschnitt 11 und im hinteren Abschnitt 13 unterschiedliche Faltungsstrukturen 14 vorgesehen sein können. Denkbar ist, im vorderen Abschnitt 11 eine U-förmige Faltungsstruktur und/oder im hinteren Abschnitt 13 eine breitere C-förmige Faltungsstruktur 14 vorzusehen. Eine weitere, mittige, longitudinale Faltungsbahn 17 kann zur Anpassung an die männliche Anatomie und/oder im hinteren Abschnitt 13 verwendet werden.

Wie beispielsweise in den Fig. 5 e) und f) dargestellt, kann vorgesehen sein, dass der absorbierende Kern im vorderen und/oder im hinteren Abschnitt 11, 13 eine weitere longitudinale Faltungsbahn 17 aufweist, die eine Verlängerung des Kanals 16 im mittleren Abschnitt 12 bildet.

Weiterhin kann vorgesehen sein, dass die longitudinalen Faltungsbahnen 17 im vorderen und/oder hinteren Abschnitt 11, 13 nur genau einmal miteinander verbunden sind. Diese Verbindung kann über die schiefen Faltungsbahnen 18 vorgesehen sein.

Auch kann vorgesehen sein, dass die longitudinalen und/oder die schiefen Faltungsbahnen 17, 18 im vorderen und/oder hinteren Abschnitt 11, 13 symmetrisch zur longitudinalen Mittellinie X angeordnet sind.

Sofern das Kanalsystem 15 mehrere Kanäle 16 aufweist, kann auch vorgesehen sein, dass diese Kanäle 16 symmetrisch zur longitudinalen Mittellinie X angeordnet sind.

Wie bereits erwähnt, ergibt sich die Definition der Abschnitte 11, 12, 13 funktional. Nichtsdestotrotz ist vorzugsweise vorgesehen, dass der mittlere Abschnitt 12 mindestens 25%, vorzugsweise mindestens 35%, der Längserstreckung des absorbierenden Kerns 4 und/oder höchstens 60%, vorzugsweise höchstens 50%, der Längserstreckung des absorbierenden Kerns 4 ausmacht. Zusätzlich oder alternativ kann vorgesehen sein, dass der vordere Abschnitt 11 und/oder der hintere Abschnitt 13 mindestens 20%, vorzugsweise mindestens 25%, und/oder höchstens 35%, vorzugsweise höchstens 30%, der Längserstreckung des absorbierenden Kerns 4 ausmacht. Vorzugsweise kann, wie ersichtlich, vorgesehen sein, dass der mittlere Abschnitt 12 jeweils an dem Knotenpunkt 19 endet.

Hier und vorzugsweise ist vorgesehen, dass der absorbierende Kern 4 im vorderen Abschnitt 11 und im hinteren Abschnitt 13 einen Bereich in Längsrichtung L aufweist, in dem keine Faltungsstrukturen 14 und Kanäle 16 vorgesehen sind.

Es kann vorgesehen sein, dass der absorbierende Kern 4 eine rechteckige Form aufweist. Wie aus den Figuren ersichtlich, ist es hier und vorzugsweise jedoch so, dass der absorbierende Kern 4 in Längsrichtung L mittig einen verjüngten Bereich aufweist. Auch dies kann den Tragekomfort erhöhen, da zwischen den Beinen weniger Platz in die Breite vorhanden ist.

In einer bevorzugten Ausführungsform sind die Faltungsstrukturen 14 und das Kanalsystem 15 in ihren Erstreckungen in Längsrichtung L und Breitenrichtung B so ausgestaltet, dass das kleinstmögliche Rechteck, das um die Faltungsstruktur oder Faltungsstrukturen 14 und das Kanalsystem 15 angeordnet werden kann (bounding box), eine Fläche von höchstens 40%, vorzugsweise höchstens 30%, und/oder mindestens 15%, vorzugsweise mindestens 20%, weiter vorzugsweise etwa 25%, der Gesamtfläche des absorbierenden Kerns 4 ausmacht.

Es kann vorgesehen sein, dass der absorbierende Kern 4 in einem Teil des vorderen Abschnitts 11 und/oder in einem Teil des hinteren Abschnitts 13 breiter entlang der Breitenrichtung B ist als in einem Teil des mittleren Abschnitts 12, vorzugsweise breiter als der breiteste Teil des mittleren Abschnitts 12. Vorzugsweise ist es außerdem so, dass der absorbierende Kern 4 in einem Abschnitt im hinteren Abschnitt 13 breiter als der breiteste Teil im vorderen Abschnitt 11 ist. Gerade bei Rückenschläfern wird so effektiver ein Auslaufen verhindert. Weiterhin ist es vorzugsweise so, dass der hintere Abschnitt 13 überall breiter als der mittlere Abschnitt 12 ist, und/oder, dass der vordere Abschnitt 11 überall breiter als der mittlere Abschnitt 12 ist, und/oder, dass der absorbierende Kern 4 im vorderen und mittleren und hinteren Abschnitt 11, 12, 13 jeweils im Wesentlichen eine konstante Breite und zwischen den unterschiedlichen Breiten Übergänge aufweist. Besondere eigenständige Relevanz kommt hier beispielsweise auch der Kombination aus unterschiedlichen Breiten und spezifischen Kapazitäten des absorbierenden Kerns 4 insbesondere in Kombination mit der Ventilfunktion zu.

Hier und vorzugsweise weist der vordere Abschnitt 11 über zumindest 50% seiner Länge, vorzugsweise mindestens 80% seiner Länge eine Breite von höchstens 110 mm und/oder mindestens 90 mm, vorzugsweise 100 mm auf. Der mittlere Abschnitt 12 weist hier und vorzugsweise über zumindest 30% seiner Länge, vorzugsweise mindestens 50% seiner Länge eine Breite von mindestens 82 mm und/oder höchstens 102 mm, vorzugsweise 92 mm auf. Der hintere Abschnitt 13 weist hier und vorzugsweise über zumindest 50% seiner Länge, vorzugsweise mindestens 80% seiner Länge eine Breite von höchstens 115 mm und/oder mindestens 95 mm, vorzugsweise 105 mm auf.

Auch die Breiten der Faltungsbahnen 17, 18 und Kanäle 16 tragen zur Funktion bei. Dabei ist es vorzugsweise so, dass die Faltungsbahnen 17, 18 im vorderen und/oder hinteren Abschnitt 11, 13 und/oder der Kanal 16 im mittleren Abschnitt 12 zumindest teilweise, insbesondere durchgehend, eine Breite von mindestens 4 mm, vorzugsweise mindestens 6 mm, und/oder höchstens 12 mm, vorzugsweise höchstens 10 mm, weiter vorzugsweise etwa 8 mm aufweist. Zusätzlich oder alternativ können die Faltungsbahnen 17, 18 im vorderen und/oder hinteren Abschnitt 11, 13 und/oder der Kanal 16 im mittleren Abschnitt 12 zumindest teilweise, insbesondere durchgehend, eine Breite von 3 % bis 15 %, vorzugsweise 5 % bis 10 %, der Breite des absorbierenden Kerns 4 an der jeweiligen Stelle aufweisen.

Die Breite der Kanäle 16 beziehungsweise Faltungsbahnen 17, 18 wird dabei senkrecht zu deren Orientierung gemessen.

Mit Blick auf Fig. 4 und wie bereits angedeutet kann vorgesehen sein, dass der absorbierende Kern außerhalb der Faltungsbahnen 17, 18 und Kanäle 16 eine Tiefe T, insbesondere eine Tiefe T der Schicht 10 mit Saugmaterial oder des gesamten absorbierenden Kerns 4, orthogonal zur Längsrichtung L und zur Breitenrichtung B aufweist und dass die Tiefe T in Breitenrichtung B konstant ist und/oder in Längsrichtung L variiert. Die Tiefe T ist dabei im vorderen Abschnitt 11 und/oder hinteren Abschnitt 13 im Mittel geringer als im mittleren Abschnitt 12. Die Kanäle 16 können genutzt werden, um Flüssigkeit zwischen Regionen mit unterschiedlicher Tiefe T zu verteilen.

Vorzugsweise ist es so, dass der absorbierende Kern 4 in Längsrichtung Zonen 20 mit unterschiedlicher spezifischer Kapazität aufweist. Bevorzugt ist dabei, dass zwischen mindestens zwei, insbesondere allen, der Zonen 20 mit unterschiedlicher spezifischer Kapazität eine Übergangszone 21 mit graduell abnehmender beziehungsweise zunehmender spezifischer Kapazität angeordnet ist. Ob die spezifische Kapazität abnimmt oder zunimmt, ist dabei nur eine Frage der Betrachtungsrichtung.

Unter dem Begriff "spezifische Kapazität" wird dabei die Kapazität des absorbierenden Kerns, gebildet insbesondere durch das Saugmaterial und insbesondere den Superabsorber, an einer Position verstanden. Diese wird gebildet durch die Füllmenge insbesondere des Superabsorbers, dessen Dichte und die Tiefe T des absorbierenden Kerns, wobei diese miteinander zusammenhängen. Ausgedrückt werden kann die spezifische Kapazität in ml/cm², wobei die Milliliter pro Quadratzentimeter Flüssigkeit gemeint sind die aufgenommen werden können.

Hier und vorzugsweise wird die Änderung der spezifischen Kapazität, insbesondere nur, durch eine Veränderung der Tiefe T erzielt. Immer wenn eine spezifische Kapazität angesprochen wird, kann in einer bevorzugten Ausführungsform damit also auch die Tiefe T gemeint sein.

Mindestens eine der Übergangszonen 21, vorzugsweise mindestens zwei der Übergangszonen 21, können sanfte Übergangszonen 21 mit einer Länge von mindestens 10 mm in Längsrichtung L, vorzugsweise mindestens 15 mm, weiter vorzugsweise mindestens 20 mm, und/oder mit einer Länge von mindestens 1%, weiter vorzugsweise mindestens 2%, weiter vorzugsweise mindestens 4%, der Länge in Längsrichtung L des absorbierenden Kerns 4 sein. Wie in Fig. 4 ersichtlich, sind die Übergangszonen 21 mit Änderungen der Faltung des absorbierenden Kerns 4 synchronisiert.

Es kann vorgesehen sein, dass mindestens eine Zone 20 jeweils dem vorderen und/oder dem hinteren und/oder dem mittleren Abschnitt 11, 13, 12 zugeordnet ist. Zusätzlich oder alternativ kann die Grenze zwischen vorderem und mittlerem Abschnitt 11, 12 und oder zwischen hinterem und mittlerem Abschnitt 13, 12 innerhalb einer, insbesondere sanften, Übergangszone 21 liegen. Auch kann vorgesehen sein, dass die mittlere spezifische Kapazität einer, insbesondere sanften, Übergangszone 21, insbesondere jeweils, in Längsrichtung L innerhalb der Längserstreckung E der vorderen und/oder hinteren Faltungsstruktur 14 liegt. Es hat sich gezeigt, dass durch diese Abstimmung zwischen Änderung der spezifischen Kapazität, insbesondere bei einer Tiefenänderung, und Faltungsänderung ein besonders hoher Tragekomfort möglich ist.

Die spezifische Kapazität im mittleren Abschnitt 12 ist vorzugsweise zumindest teilweise und/oder im Mittel, insbesondere durchgehend, größer als in Teilen des vorderen und/oder hinteren Abschnitts 11, 13 vorzugsweise als das Mittel der spezifischen Kapazitäten des vorderen und/oder hinteren Abschnitts 11, 13 und/oder des jeweils gesamten Abschnitts 11, 13. Für das Flüssigkeitsmanagement und den Übergang der Faltung zusammen hat sich herausgestellt, dass es vorteilhaft ist, wenn der Kanal 16 in mindestens oder genau eine, vorzugsweise in mindestens oder genau zwei, insbesondere sanfte, Übergangszonen 21 reicht und vorzugsweise darin endet.

Weiterhin ist es vorzugsweise so, dass der absorbierende Kern 4 im vorderen Abschnitt 11 und/oder im hinteren Abschnitt 13 über mindestens 50%, vorzugsweise mindestens 70%, der Fläche des Abschnitts 11, 13 eine spezifische Kapazität aufweist, die geringer ist als eine spezifische Kapazität, die der absorbierende Kern 4 über mindestens 50%, vorzugsweise mindestens 70%, der Fläche im mittleren Abschnitt 12 aufweist. Vorzugsweise ist eine, insbesondere sanfte, Übergangszone 21 zwischen den Bereichen unterschiedlicher spezifischer Kapazitäten im mittleren Abschnitt 12 und im vorderen und/oder hinteren Abschnitt 11, 13 zumindest 50%, vorzugsweise mindestens 80%, Längsrichtung L im Bereich der Faltungsstruktur 14 im vorderen Abschnitt 11 beziehungsweise hinteren Abschnitt 13 angeordnet.

Es ist also so, dass vorzugsweise, abgesehen von den Übergangszonen 21, je Abschnitt 11, 12, 13 je eine Zone 20 mit im Wesentlichen konstanter spezifischer Kapazität vorgesehen ist. Die Übergangszonen 21 liegen vorzugsweise vollständig im Bereich der Längserstreckung E der Faltungsstruktur 14.

Auch der Kanal 16 bildet vorzugsweise eine Faltungsbahn aus. Diese hat vorzugsweise eine stabilisierende Wirkung auf die Schicht 10 mit Saugmaterial, so dass der Kanal eine stabilisierende Faltungsbahn bildet. Die stabilisierende Wirkung verhindert, dass das Saugmaterial klumpt, abreißt oder sich stellenweise sammelt. Insbesondere auf Grund der größeren spezifischen Kapazität im mittleren Abschnitt 12 ist dies vorteilhaft.

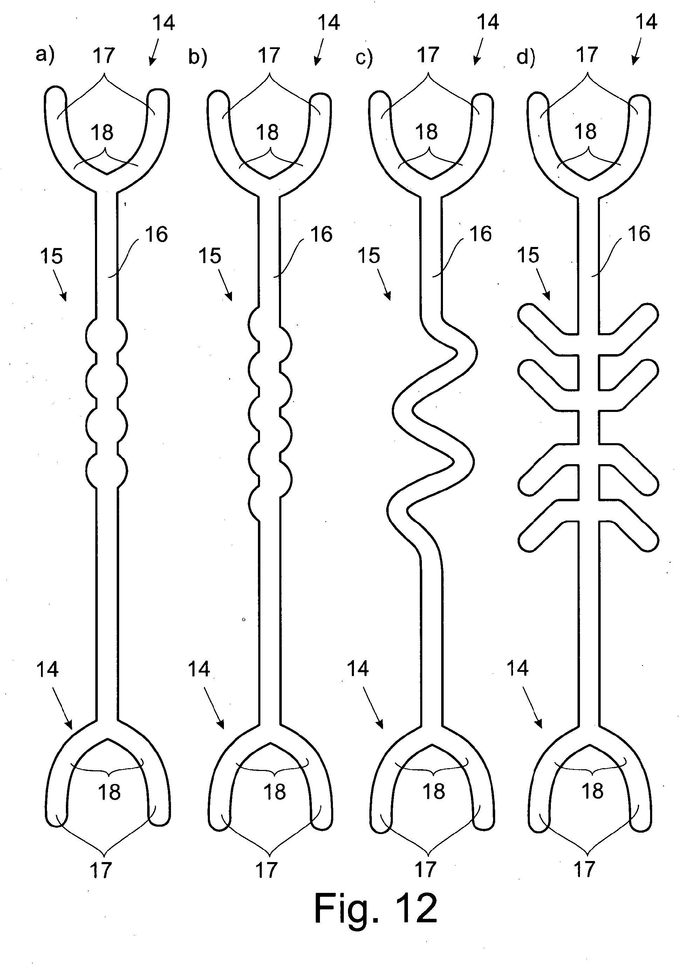

Der mindestens eine Kanal 16 im mittleren Abschnitt 12 verläuft vorzugsweise immer zumindest teilweise innerhalb eines Bereichs der mittleren 15%, vorzugsweise der mittleren 10%, der Breite des absorbierenden Kerns 4. Der Begriff "immer" meint in diesem Fall, dass im gesamten mittleren Abschnitt 12, soweit der Kanal 16 dort vorhanden ist, der Kanal mit irgendeinem Abschnitt seiner Breite innerhalb des entsprechenden Bereichs verläuft. Vorzugsweise verläuft er mit einem Rand oder vollständig, also mit beiden Rändern, im jeweiligen Bereich. Diesbezüglich wird angemerkt, dass dies in den Figuren, insbesondere in Fig. 12 c) nicht immer der Fall ist.

Hinsichtlich der Fig. 12 ist es vorzugsweise so, dass das Kanalsystem 15, insbesondere der Kanal 16, im mittleren Abschnitt 12 teilweise Verbreiterungen oder nach außen laufende Abschnitte aufweist. Dadurch wird eine bessere Flüssigkeitsaufnahme in der Schicht 10 mit Saugmaterial erreicht. Zusätzlich können im Kanal 16 wie in Fig. 12 c) gezeigt, Kurven vorgesehen sein, die eine Fließgeschwindigkeit der Flüssigkeit verringern und gleichzeitig eine bessere Verteilung erzeugen.

Wie in Fig. 7 gezeigt, kann vorgesehen sein, dass der absorbierende Kern 4 weitere Faltungsbahnen 22 aufweist, die vorzugsweise keine Kanäle sind. Diese können insbesondere als Prägelinien ausgestaltet sein, die, wie in Fig. 8 sichtbar, nicht vollständig durch die Schicht 10 mit Saugmaterial gehen. Diese können zum einen aus optischen Gründen vorgesehen sein, zum anderen jedoch auch die Faltung des absorbierenden Kerns 4 unterstützen. Insbesondere kann auch die Faltungsstruktur 14, vorzugsweise nur hinten oder nur vorne, vollständig oder teilweise, aus Prägelinien gebildet sein.

Die weiteren Faltungsbahnen 22 können anatomisch auf die Passform des Hygieneartikels 1 abgestimmt sein. Sie verlaufen hier und vorzugsweise zumindest teilweise parallel zu den Faltungsbahnen 17, 18 der Faltungsstruktur 14 im vorderen und/oder hinteren Abschnitt 11, 13 und/oder zu dem Kanal 16. Es kann vorgesehen sein, dass der absorbierende Kern 4 im mittleren Abschnitt 12 in Längsrichtung 11 verlaufende weitere, äußere Faltungsbahnen 22 aufweist, die vorzugsweise in Breitenrichtung B in den äußeren Dritteln des absorbierenden Kerns 4 angeordnet sind. Die weiteren Faltungsbahnen 22 können symmetrisch zur longitudinalen Mittellinie X angeordnet sein.

Grundsätzlich kann vorgesehen sein, dass der absorbierende Kern 4 nur die longitudinalen und schiefen Faltungsbahnen 17, 18 und den Kanal 16 als Kanäle aufweist, und/oder, dass alle Kanäle 16 des absorbierenden Kerns 4 miteinander verbunden sind.

Der Fokus der Betrachtung soll nun von den Kanälen 16 weg hin zu der Schicht 10 mit Saugmaterial im Übrigen gehen. Diese Betrachtung betrifft die vorschlagsgemäße Lehre und bildet teilweise einen anderen Blickwinkel auf die obigen Ausführungen und teilweise eigene, unabhängige Gesichtspunkte ab.

Im Sinne der vorschlagsgemäßen Lehre kann vorgesehen sein, dass die Schicht 10 mit Saugmaterial durch die Kanäle 16 in Breitenrichtung in Saugkanäle 23 getrennt wird, dass die Schicht 10 mit Saugmaterial in einem Abschnitt entlang der Längsrichtung L genau drei Saugkanäle 23 aufweist und in einem Abschnitt genau zwei Saugkanäle 23, vorzugsweise dass die Schicht 10 mit Saugmaterial in einem Abschnitt entlang der Längsrichtung L genau einen Saugkanal 23 aufweist.

Die Betrachtung der Saugkanäle 23 zeigt, dass durch die Verteilung von Kanälen, oder allgemeiner Bahnen 16, 17, 18, 18a, mit einer Flüssigkeitsleitcharakteristik, die von der der Saugkanäle 23 abweicht, die Schicht 10 mit Saugmaterial getrennt werden kann. Dieses Trennen führt neben der verbesserten Faltbarkeit zu unterschiedlichen Flüssigkeitsverteilungen. Natürlich war diese unterschiedliche Flüssigkeitsverteilung auch bei bekannten Kanälen bereits vorhanden, erst eine dedizierte Betrachtung der einzelnen Saugkanäle 23, die an der menschlichen Anatomie gespiegelt ist, führt jedoch dazu, dass der Tragekomfort erhöht werden kann. Dem liegt die Überlegung zu Grunde, dass ein Hygieneartikel 1 beziehungsweise ein absorbierender Kern 4 selten vollständig mit Flüssigkeit gefüllt ist. Ein wenig gefüllter Zustand hingegen muss häufig noch einige Zeit getragen werden. Wird diese wenige Flüssigkeit bewusst verteilt, erhöht sich der Tragekomfort.

Die vorschlagsgemäße Lehre betrifft einen absorbierenden Kern 4 für einen Hygieneartikel 1, insbesondere für eine Windel, wobei der absorbierende Kern 4 eine Längsrichtung L entlang einer Längsachse, eine Breitenrichtung B, ein körperseitiges Deckblatt 8, ein körperfernes Rückenblatt 9 und eine zwischen dem Deckblatt 8 und dem Rückenblatt 9 angeordnete Schicht 10 mit Saugmaterial, vorzugsweise umfassend Superabsorber, aufweist, wobei die Schicht 10 mit Saugmaterial Saugkanäle 23 mit einer Flüssigkeitsleitcharakteristik aufweist, wobei die Schicht 10 mit Saugmaterial durch Bahnen 16, 17, 18, 18a, insbesondere Kanäle, mit einer abweichenden Flüssigkeitsleitcharakteristik in die Saugkanäle 23 geteilt ist, wobei die Schicht 10 mit Saugmaterial in einem ersten Abschnitt 24 entlang der Längsrichtung L mindestens drei Saugkanäle 23 aufweist und in einem zweiten Abschnitt 25 weniger Saugkanäle 23 als im ersten Abschnitt 24 jedoch mindestens zwei Saugkanäle 23 aufweist.

Bezüglich der Ausgestaltung, insbesondere Form, der Bahnen 16, 17, 18, 18a darf auf alle Ausführungen zu den Kanälen 16 und den Faltungsbahnen 17, 18 verwiesen werden.

Die Bahnen entsprechen insbesondere den bereits ausführlich beschriebenen Kanälen. Genauso denkbar wäre jedoch, dass sie in Querrichtung und/oder in Längsrichtung eine höhere oder eine geringere Flüssigkeitsleitgeschwindigkeit aufweisen als die Schicht 10 mit Saugmaterial im Übrigen. Anstelle von Kanälen könnten also auch Flüssigkeitssperren oder Flüssigkeitsverteiler in Querrichtung vorgesehen sein.

Wesentlich nach der vorschlagsgemäßen Lehre ist nun, dass der absorbierende Kern 4, insbesondere die Schicht 10 mit Saugmaterial, in Längsrichtung L hintereinander angeordnete Zonen 20 mit unterschiedlicher spezifischer Kapazität aufweist und dass der absorbierende Kern 4 in dem ersten Abschnitt zumindest teilweise unterschiedliche spezifische Kapazitäten aufweist. Durch die Kombination von unterschiedlichen Anzahlen an Zonen 20 mit unterschiedlicher spezifischer Kapazitäten und unterschiedlichen Anzahlen an Saugkanälen 23 wird auf besonders interessante Art und Weise die Möglichkeit geschaffen, unterschiedliche Saugkanäle 23 flexibel mit unterschiedlichen Flüssigkeitsmengen zu füllen und darauf herstellungstechnisch mit unterschiedlichen Mengen an Saugmaterial zu reagieren.

Für vorschlagsgemäße Lehre wird grundsätzlich auf alle Ausführungen zu der ersten Lehre verwiesen. Explizit wird hier zusätzlich auf die Ausführungen zu den Zonen 20 mit unterschiedlicher T und den Übergangszonen 21 verwiesen.

Der erste Abschnitt 24 und der zweite Abschnitt 25 müssen nicht mit den Abschnitten 11, 12, 13 korrespondieren.

Es kann vorgesehen sein, dass der erste Abschnitt 24 zumindest teilweise in einer, insbesondere sanften, Übergangszone 21 liegt. Vorzugsweise kann vorgesehen sein, dass eine, insbesondere sanfte, Übergangszone 21 vollständig in dem ersten Abschnitt 24 liegt. Dabei ist jeweils die Anordnung in Längsrichtung L gemeint. Diesbezüglich wird auch auf Fig. 4 verwiesen.

Es kann vorgesehen sein, dass die Schicht 10 mit Saugmaterial in dem ersten Abschnitt 24 genau drei Saugkanäle 23 und/oder in dem zweiten Abschnitt 25 genau zwei Saugkanäle 23 aufweist, vorzugsweise, dass die Schicht 10 mit Saugmaterial in einem Abschnitt entlang der Längsrichtung L genau einen Saugkanal 23 aufweist.

Weiterhin kann vorgesehen sein, dass mindestens ein Reserve-Saugkanal 26 in Längsrichtung L auf einer, vorzugsweise zur Mitte des absorbierenden Kerns 4 orientierten, Seite durch eine in Breitenrichtung B verlaufende Bahn 18, 18a von den anderen Saugkanälen 12 getrennt ist. Vorzugsweise ist der Reservesaugkanal 26, gegebenenfalls jeweils ein Reserve-Saugkanal 26, im vorderen und/oder hinteren Drittel, insbesondere Viertel, des absorbierenden Kerns 4 angeordnet.

Zusätzlich oder alternativ kann der der Reserve-Saugkanal 26 oder jeweils ein Reserve-Saugkanal 26 im vorderen und/oder hinteren Abschnitt 11, 13 angeordnet sein. Bezüglich der in Breitenrichtung verlaufenden Bahn 18, 18a reicht es aus, wenn diese, wie aus den Figuren ersichtlich, nur teilweise in Breitenrichtung B verläuft. Interessant ist hier, dass sich herausgestellt hat, dass auch bei den vorgesehenen Kanälen 16 die Flüssigkeit häufig am äußeren Rand der Kanäle 16 entlangfließt, sofern nur moderate Mengen Flüssigkeit aufgebracht werden. Dadurch kann bei einem absorbierenden Kern 4 mit Kanälen 16 und insbesondere einer Faltungsstruktur 14 aus Kanälen 16 einfach ein Reserve-Saugkanal 26 vorgesehen werden. Dieser wird in vielen Fällen relativ trocken bleiben und so den Tragekomfort deutlich erhöhen. Nichtsdestotrotz steht er in Notfällen zur Verfügung.

Das gleiche Prinzip kann auch auf mehrere Reserve-Saugkanäle 26 angewendet werden. Vorzugsweise sind im vorderen und/oder hinteren Drittel, insbesondere Viertel und/oder im vorderen Abschnitt 11 und/oder hinteren Abschnitt 13 mindestens ein, vorzugsweise jeweils mindestens zwei, vorzugsweise mindestens drei, Reserve-Saugkanäle 26 angeordnet.

Vorzugsweise ist vorgesehen, dass der absorbierende Kern 4 in Längsrichtung L mindestens fünf, vorzugsweise genau fünf, Abschnitte mit jeweils zu den in Längsrichtung L benachbarten Abschnitten variierende Anzahl an Saugkanälen 23 aufweist. Zusätzlich oder alternativ kann vorgesehen sein, dass der absorbierende Kern 4 entlang der Längsrichtung L erst drei Saugkanäle 23, dann zwei Saugkanäle 23, dann drei Saugkanäle 23 aufweist. Vorzugsweise weist der absorbierende Kern 4 entlang der Längsrichtung L erst einen Saugkanal 23, dann drei Saugkanäle 23, dann zwei Saugkanäle 23, dann drei Saugkanäle 23, dann einen Saugkanal 23 auf.

Im Folgenden soll nunmehr auf die Kombination der Saugkanäle 23 mit den variierenden spezifischen Kapazitäten eingegangen werden. Dabei ist bevorzugt vorgesehen, dass die drei Saugkanäle 23 des ersten Abschnitts 24 jeweils in einer Übergangszone 21 zwischen einem Bereich größerer spezifischer Kapazität in Längsrichtung L in Richtung der Mitte des absorbierenden Kerns 4 und einem Bereich mit geringerer spezifischer Kapazität angeordnet sind. Der Bereich mit größerer spezifischer Kapazität der von der Übergangszone 21 aus gesehen in Richtung der Mitte des absorbierenden Kerns 4 angeordnet ist, kann insbesondere Teil des mittleren Abschnitts 12 sein. Zusätzlich kann vorgesehen sein, dass die drei Saugkanäle 23 des ersten Abschnitts 24 zusätzlich in dem Bereich mit geringerer spezifischer Kapazität angeordnet sind, sich also der Übergangszone 21 dorthin erstrecken.

Der Bereich mit größerer spezifischer Kapazität weist vorzugsweise eine Tiefe T von 3 bis 6 mm, vorzugsweise 4 bis 5 mm, auf. Die Bereiche mit geringer spezifischer Kapazität weisen vorzugsweise eine Tiefe T von 1 bis 3 mm, vorzugsweise etwa 2 mm, auf.

Die zwei Saugkanäle 23 des zweiten Abschnitts 25 sind vorzugsweise in dem Bereich mit größerer spezifischer Kapazität angeordnet. Weiter vorzugsweise sind die Bereiche mit einem Saugkanal 23 in den Bereichen mit geringerer spezifischer Kapazität angeordnet.

Es kann vorgesehen sein, dass die Schicht 10 mit Saugmaterial in einem Abschnitt der vorderen und/oder hinteren 40%, vorzugsweise 30%, der Länge des absorbierenden Kerns 4 mindestens drei, vorzugsweise genau drei Saugkanäle 23 aufweist, und/oder, dass die Schicht 10 mit Saugmaterial in einem Teil der mittleren 50%, vorzugsweise der mittleren 30%, weiter vorzugsweise der mittleren 10%, und/oder in mindestens den mittleren 10%, vorzugsweise mindestens den mittleren 20%, höchstens zwei, vorzugsweise genau zwei Saugkanäle aufweist. So kann in Längsrichtung des absorbierenden Kerns 4 eine hohe Flüssigkeitsaufnahme mit einer guten Stabilität vereint werden. Diese Funktion wurde grundsätzlich bereits zur ersten Lehre erläutert.

Die entstehende Flüssigkeitsverteilung des absorbierenden Kerns ermöglicht es, den Tragekomfort durch Reduktion der Breite des absorbierenden Kerns 4 zu erhöhen. Vorzugsweise ist es so, dass der absorbierende Kern 4 mindestens zwei unterschiedliche Breiten aufweist, vorzugsweise, dass der absorbierende Kern 4 einen Bereich 27 von mindestens 10% der Länge des absorbierenden Kerns 4 mit geringerer Breite aufweist und mindestens zwei, in Längsrichtung L beidseits des Bereichs mit geringerer Breite 27 angeordnete, Bereiche 28 von mindestens 15% der Länge des absorbierenden Kerns 4 mit größerer Breite.

Der Bereich mit geringerer Breite 27 umfasst vorzugsweise die Mitte des absorbierenden Kerns 4, insbesondere die transversale Mittellinie Y. Zusätzlich oder alternativ umfassen die Bereiche 28 mit größerer Breite vorzugsweise die Enden in Längsrichtung L des absorbierenden Kerns 4 oder reichen mindestens 10% der Länge des absorbierenden Kerns 4 an diese heran. Weiterhin kann jeweils ein Bereich 29 mit sich graduell verändernder Breite zwischen dem Bereich 27 mit geringerer Breite und den Bereichen 28 mit größerer Breite angeordnet sein.

In den Bereichen mit geringerer Breite 27 oder größerer Breite 28 ist die Breite vorzugsweise konstant oder weicht um maximal 10%, vorzugsweise um maximal 5% von ihrem Mittelwert ab. Vorzugsweise sind genau fünf Breitenbereiche 27, 28, 29, inklusive der Bereiche 29 mit sich graduell ändernder Breite vorgesehen. Zu den bevorzugten Breiten des absorbierenden Kerns 4 wird wieder nach oben verwiesen. Es ist hier und vorzugsweise so, dass in dem Bereich 27 mit geringerer Breite 27 die spezifische Kapazität des absorbierenden Kerns 4, zumindest teilweise, vorzugsweise zu mindestens 60%, weiter vorzugsweise zu mindestens 80%, der Fläche, weiter vorzugsweise vollständig, größer ist als über mindestens 50%, vorzugsweise mindestens 80%, weiter vorzugsweise 100%, der Fläche eines oder beider Bereiche 28 mit größerer Breite.

In einer weiteren bevorzugten Ausführungsform weist der absorbierende Kern 4 in dem Bereich 27 mit geringerer Breite, insbesondere in dem gesamten Bereich 27, genau zwei Saugkanäle 23 auf. Zusätzlich oder alternativ kann in dem Bereich 28 mit größerer Breite der absorbierende Kern 4 abschnittsweise genau einen, und vorzugsweise abschnittsweise genau drei, Saugkanal 23 beziehungsweise Saugkanäle 23 aufweisen.

Zu den diversen möglichen Formen der Bahnen 16, 17, 18, 18a wird auf die Formen der Faltungsstruktur 14 und des Kanals 16, die jeweils teilweise oder vollständig durch Bahnen 16, 17, 18, 18a ersetzt werden können, verwiesen.

Vorzugsweise ist es dabei so, dass der absorbierende Kern 4 eine Bahn 16 aufweist, die den absorbierenden Kern 4 in zwei Saugkanäle 23 teilt, die durch die mittleren 20%, vorzugsweise die mittleren 35% der Länge des absorbierenden Kerns 4 verläuft. Weiter bevorzugt verläuft die Bahn 16 währenddessen zumindest abschnittsweise, vorzugsweise durchgehend, in den mittleren 20%, vorzugsweise den mittleren 10%, der Breite des absorbierenden Kerns 4. Die Vorteile der so entstehenden anatomischen Faltung wurden bereits ausführlich diskutiert.

Es kann vorgesehen sein, dass sich an die eine Bahn 16 im vorderen und/oder im hinteren Teil, insbesondere im vorderen und/oder hinteren Abschnitt 11, 13, des absorbierenden Kerns 4 zwei Bahnen 17, 18, 18a in Form auseinanderlaufende Arme, insbesondere U-förmig oder C-förmig oder mit der einen Bahn 16 T-förmig, anschließen. Dafür wird insbesondere auf die Erläuterungen zu den schiefen Faltungsbahnen 18 oder den transversalen Faltungsbahnen 18a verwiesen. Vorzugsweise ist es dabei so, dass die zwei Bahnen 17, 18, 18a im vorderen und/oder hinteren Teil des absorbierenden Kerns 4 ausgehend von der einen Bahn in der Übergangszone 21 und/oder einem Bereich 29 mit sich graduell verändernder Breite 29 beginnen und/oder enden oder sich hindurch erstrecken.

Wie bereits erwähnt, kann vorgesehen sein, dass die Bahnen 16, 17, 18, 18a Kanäle sind, die zumindest in Längsrichtung L eine schnellere Flüssigkeitsleitung als die Saugkanäle 23 aufweisen, und/oder, dass die Bahnen 16, 17, 18, 18a kein oder im Wesentlichen kein Saugmaterial, insbesondere keinen Superabsorber, aufweisen.

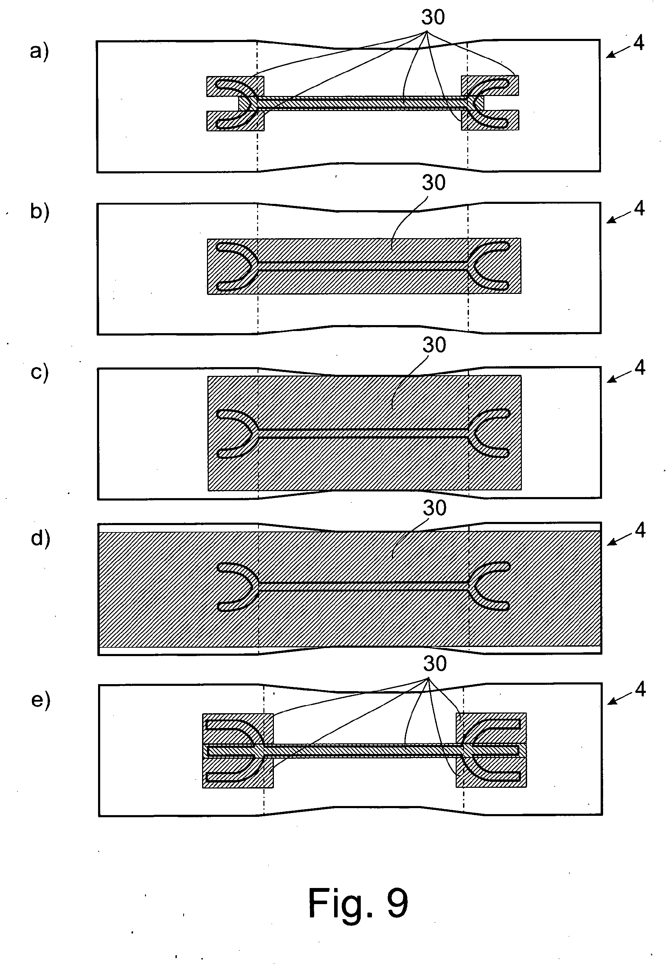

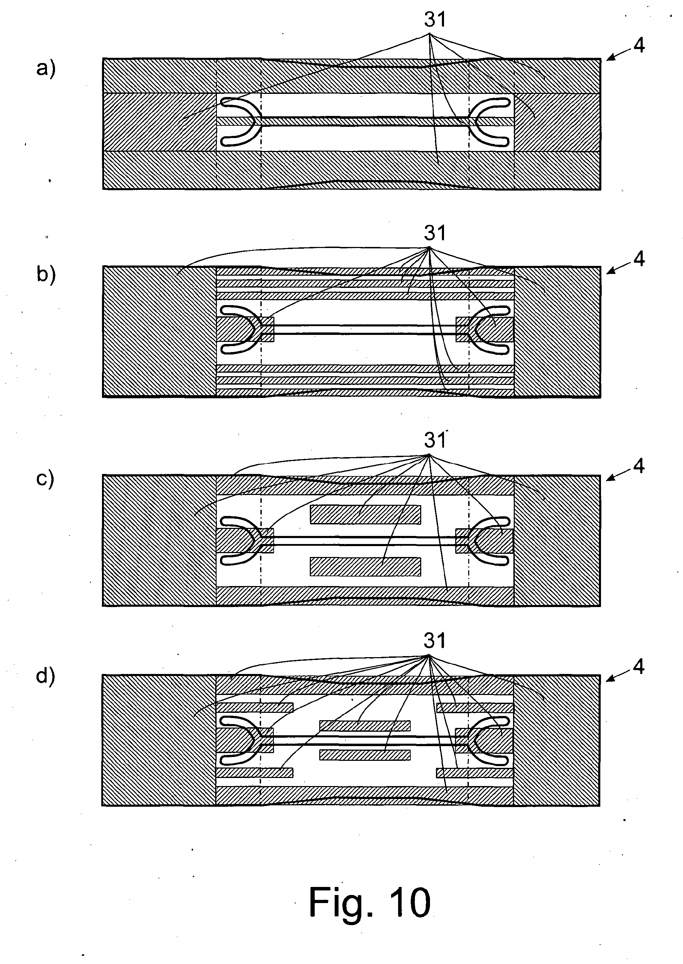

Mit Blick auf die Fig. 9 und 10 soll nun erläutert werden, wie der absorbierende Kern 4 vorzugsweise zu einem Laminat befestigt, insbesondere verklebt, wird. Fig. 9 betrifft dabei bevorzugte Verbindungen zwischen dem Deckblatt 8 und der Schicht 10 mit Saugmaterial und Fig. 10 zwischen der Schicht 10 mit Saugmaterial und dem Rückenblatt 9.

Zur Befestigung kann jeweils Kleber, insbesondere Heißkleber, eingesetzt werden. Genauso bevorzugt können Ultraschallschweißen, Hitzeprägungen und andere kleberfreie Varianten verwendet werden. Sofern Kleber verwendet wird, können hierfür Schlitzdüsen, Sprühdüsen, Runddüsen, ein Walzenauftrag und dergleichen verwendet werden. Der Kleber kann in Mustern wie Spiralen, Streifen, Punkten oder als Sprühnetz aufgetragen werden. Zur Verbindung zwischen dem Deckblatt 8 und der Schicht 10 mit Saugmaterial und dem Rückenblatt 9 kann ein erstes Verbindungsmuster 30 vorgesehen sein. Beispiele dafür sind gezeigt. Das erste Verbindungsmuster 30 kann bei Verwendung von Kleber ein Kleberauftrag sein.

Das erste Verbindungsmuster 30 kann an den Kanälen 16 und/oder der Verbindungsstruktur 14 und/oder den Verbindungsstrukturen 14 orientiert sein. Es kann mit diesem zumindest 50% überlappen oder großflächiger aufgebracht sein. Es kann aus ein oder mehr Rechtecken im Bereich der jeweiligen Verbindungsstruktur 14 und einem weiteren Rechteck im Bereich des Kanals 16 bestehen. Es kann auch ganzflächig oder nahezu ganzflächig auf das Deckblatt 8 aufgebracht werden. Es kann grundsätzlich vor dem Aufbringen der Schicht 10 mit Saugmaterial auf das Deckblatt 8 aufgebracht werden.

Auf die Schicht 10 mit Saugmaterial oder insbesondere das Rückenblatt 8 kann ein zweites Verbindungsmuster 31 aufgebracht werden. Beispiele dafür sind in Fig. 10 dargestellt. Auch das zweite Verbindungsmuster 31 kann Rechtecke umfassen. Es kann entlang und/oder außerhalb der Verbindungsstrukturen 14 und/oder Kanäle 16 aufgebracht werden. Insbesondere wird ein Bereich mit einem Saugkanal großflächig abgedeckt. Es kann teilweise oder vollständig komplementär zum ersten Verbindungsmuster 30 sein.

Nach einer weiteren Lehre, der eigenständige Bedeutung zukommt, wird ein Hygieneartikel 1, insbesondere eine Windel, mit einem vorschlagsgemäßen absorbierenden Kern 4 vorgeschlagen. Auf alle Ausführungen zu dem absorbierenden Kern 4 darf verwiesen werden.

Der Hygieneartikel 1 weist vorzugsweise ein körperseitiges Topsheet 2 und ein körperfernes Backsheet 3 auf, die vorzugsweise jeweils eine Vliesschicht aufweisen. Der absorbierende Kern 4 ist vorzugsweise zwischen dem Topsheet 2 und dem Backsheet 3 angeordnet. Vorzugsweise ist das Backsheet 3 atmungsaktiv. Vorzugsweise weist der Hygieneartikel 1 Befestigungslaschen 6 auf, die im angelegten Zustand des Hygieneartikels 1 an dem Backsheet 3 oder einer mit dem Backsheet 3 verbundenen Befestigungsfläche 32 befestigt werden können.

Neben den bereits genannten Materialien für das Topsheet 2 kann dieses auch ein Laminat aus Vlies und einer gelochten und/oder perforierten Folie umfassen. Dabei ist die gelochte und/oder perforierte Folie vorzugsweise auf der körperfernen Seite des Topsheet 2 angeordnet. Durch die Perforierungsrichtung kann das Topsheet 2 klappenähnlich ausgestaltet sein und in die vom Körper wegweisende Richtung besser flüssigkeitsdurchlässig sein. Das gleiche Material kann zusätzlich oder alternativ für die Verteilerlage 5 verwendet werden.

In Fig. 3 ist der Schichtaufbau eines bevorzugten Hygieneartikels 1, in Form einer Windel, dargestellt. Nicht alle diese Schichten müssen vorhanden sein. Vorzugsweise weist der Hygieneartikel Stehbündchen 33, insbesondere als oberste Schicht, auf. Diese dienen als innere Nässesperre und zur gezielten Nässeableitung in Richtung des absorbierenden Kerns 4.

Während vorliegend dargestellt ist, dass Topsheet 2, Backsheet 3, Verteilerlage 5, Deckblatt 8 und Rückenblatt 9 als separate Schichten vorhanden sind, kann alternativ auch vorgesehen sein, dass einige dieser Lagen durch eine gemeinsame Schicht gebildet werden. So können zum Beispiel das Rückenblatt 9 und das Backsheet 3 zusammen eine einzige Schicht sein. In einer bevorzugten Ausführungsform sind zumindest Topsheet 2, Backsheet 3, Deckblatt 8 und Rückenblatt 9 separat vorhanden.

Weiterhin kann vorgesehen sein, dass der Hygieneartikel 1 Beinöffnungen mit Beinbündchen 34 aufweist, vorzugsweise, dass die Beinöffnungen untere und obere Beinbündchen 34 aufweisen, weiter vorzugsweise, dass die Beinbündchen 34, insbesondere die unteren oder/oder oberen Beinbündchen 34, ein bis vier, vorzugsweise zwei oder drei, elastische Fäden mit einer Feinheit von 280 dtex bis 1000 dtex, vorzugsweise 480 dtex bis 800 dtex, weiter vorzugsweise 620 dtex bis 680 dtex aufweisen. Weiterhin kann vorgesehen sein, dass der Hygieneartikel 1 ein Hüftbändchen 35 zur besseren Anlage im Hüftbereich aufweist.

Im eingebauten Zustand des absorbierenden Kerns 4 in dem Hygieneartikel 1 kann vorgesehen sein, dass die Befestigungslaschen 6 und/oder die Seitenflügel 7 und/oder vordere Befestigungsohren 36 (front ears) im Verhältnis zum absorbierenden Kern 4 so angeordnet sind, dass eine Verlängerung einer gedachten Linie, ausgehend vom Knotenpunkt 19, entlang des Winkels β, den die mindestens eine schiefe Faltungsbahnen 18 gegenüber der longitudinalen Mittellinie X aufweist, die jeweilige Befestigungslasche 6 und/oder den jeweiligen Seitenflügel 7 und/oder das jeweilige vordere Befestigungsohr 36 schneidet. Die gedachte Linie läuft also ausgehend von dem Knotenpunkt 19 parallel zu dem Abschnitt der schiefen Faltungsbahn 18, der den Winkel β zwischen 30° und 60° aufweist. In Fig. 4 korrespondiert die Gedachte Linie zu der gestrichelten Linie, die den Winkel β begrenzt.

Alternativ kann vorgesehen sein, dass mindestens eines der Elemente 6, 7, 36 vollständig von dem Schnittpunkt der gedachten Linie mit dem Rand des absorbierenden Kerns 4 aus in Längsrichtung L weiter außen angeordnet ist.

Nach einer weiteren Lehre, der eigenständige Bedeutung zukommt, wird ein Herstellungsverfahren für einen Hygieneartikel mit einem vorschlagsgemäßen absorbierenden Kern 4 vorgeschlagen. Auf alle Ausführungen zu dem absorbierenden Kern 4 darf verwiesen werden.

Relevant bei dem Herstellungsverfahren ist vor allem, dass sich herausgestellt hat, dass das Abbürsten eines Volumeneinsatzes, der zum Erstellen der Kanäle 16 und/oder Faltungsbahnen 17, 18 genutzt wird, gleichzeitig zum Angleichen der Tiefe T des Saugmaterials genutzt werden kann und so neben der Verwendung von Unterdruck eine kostengünstige und effektive Variante zum Herstellen der Kanäle 16 und/oder Faltungsbahnen 17, 18 bereitstellt.

Im Einzelnen wird für das Herstellungsverfahren vorgeschlagen, dass das körperseitige Deckblatt 8 oder das körperferne Rückenblatt 9 in eine mit einer Unterdruckquelle verbundene Form eingelegt wird, dass die Form mindestens einen Volumeneinsatz in Form Bahnen 16, 17, 18, 18a aufweist, dass auf das Deckblatt 8 oder das Rückenblatt 9 Saugmaterial aufgebracht wird, dass im Bereich des Volumeneinsatzes weniger oder kein Saugmaterial aufgebracht wird oder das Saugmaterial im Bereich des Volumeneinsatzes von der Unterdruckquelle nicht oder weniger angesogen wird und so nicht oder weniger im Bereich des Volumeneinsatzes haften bleibt, vorzugsweise, dass die Form mittels einer Bürste abgebürstet wird, wodurch die Tiefe T des Saugmaterials homogenisiert und der Volumeneinsatz, zumindest großteils, insbesondere vollständig, von Saugmaterial befreit wird, dass das Deckblatt 8, ggf. das Saugmaterial und das Rückenblatt 9 miteinander verbunden, insbesondere verklebt werden.

Die Bürste ist insbesondere eine rotierende Bürste, die einen Abstand von 1 mm bis 5 mm, vorzugsweise bis 3 mm, weiter vorzugsweise bis 2 mm zu dem Volumeneinsatz aufweist.

Es ist also möglich, im Bereich des Volumeneinsatzes entweder weniger Saugmaterial aufzutreiben, oder dieses in der gleichen Menge aufzutragen und die Unterdruckquelle so einzusetzen, dass dort weniger oder kein Saugmaterial haften bleibt.

Die Faltungsstruktur 14 kann alternativ auch geprägt oder verdichtet werden oder mit anderen bekannten Methoden hergestellt.

## Patentansprüche

1. Absorbierender Kern für einen Hygieneartikel (1), insbesondere für eine Windel,
wobei der absorbierende Kern (4) eine Längsrichtung (L), eine Breitenrichtung (B), ein körperseitiges Deckblatt (8), ein körperfernes Rückenblatt (9) und eine zwischen dem Deckblatt (8) und dem Rückenblatt (9) angeordnete Schicht (10) mit Saugmaterial, vorzugsweise umfassend Superabsorber, aufweist,
wobei die Schicht (10) mit Saugmaterial Saugkanäle (23) mit einer Flüssigkeitsleitcharakteristik aufweist,
wobei die Schicht (10) mit Saugmaterial durch Bahnen (16, 17, 18, 18a), insbesondere Kanäle, mit einer abweichenden Flüssigkeitsleitcharakteristik in die Saugkanäle (23) geteilt ist,
wobei die Schicht (10) mit Saugmaterial in einem ersten Abschnitt (24) entlang der Längsrichtung (L) mindestens drei Saugkanäle (23) aufweist und in einem zweiten Abschnitt (25) weniger Saugkanäle (23) als im ersten Abschnitt (24), jedoch mindestens zwei Saugkanäle (23), aufweist,
**dadurch gekennzeichnet,**
**dass** der absorbierende Kern (4), insbesondere die Schicht (10) mit Saugmaterial, in Längsrichtung (L) hintereinander angeordnete Zonen (20) mit unterschiedlicher spezifischer Kapazität aufweist und dass der absorbierende Kern (4) in dem ersten Abschnitt (24) und in dem zweiten Abschnitt (25) zumindest teilweise unterschiedliche spezifische Kapazitäten aufweist.

2. Absorbierender Kern nach Anspruch 1, **dadurch gekennzeichnet, dass** zwischen mindestens zwei, insbesondere allen, der Zonen (20) mit unterschiedlicher spezifischer Kapazität eine Übergangszone (21) mit graduell abnehmender oder zunehmender spezifischer Kapazität angeordnet ist, vorzugsweise, dass mindestens eine der Übergangszonen (21), vorzugsweise mindestens zwei Übergangszonen (21), sanfte Übergangszonen (21) mit einer Länge von mindestens 10 mm, vorzugsweise mindestens 15 mm, weiter vorzugsweise mindestens 20 mm, und/oder mit einer Länge von mindestens 1%, weiter vorzugsweise mindestens 2%, weiter vorzugsweise mindestens 4%, der Länge des absorbierenden Kerns (4) sind.

3. Absorbierender Kern nach Anspruch 2, **dadurch gekennzeichnet, dass** der erste Abschnitt (24) zumindest teilweise in einer, insbesondere sanften, Übergangszone (21) liegt, vorzugsweise, dass eine, insbesondere sanfte, Übergangszone (21) vollständig in dem ersten Abschnitt (24) liegt.

4. Absorbierender Kern nach einem der vorhergehenden Ansprüche, **dadurch gekennzeichnet, dass** die Schicht (10) mit Saugmaterial in dem ersten Abschnitt (24) genau drei Saugkanäle (23) und/oder in dem zweiten Abschnitt (25) genau zwei Saugkanäle (23) aufweist, vorzugsweise, dass die Schicht (10) mit Saugmaterial in einem Abschnitt (24) entlang der Längsrichtung (L) genau einen Saugkanal (23) aufweist.

5. Absorbierender Kern nach einem der vorhergehenden Ansprüche, **dadurch gekennzeichnet, dass** mindestens ein Reserve-Saugkanal (26) in Längsrichtung (L) auf einer, vorzugsweise zur Mitte des absorbierenden Kerns (4) orientierten, Seite durch eine in Breitenrichtung verlaufende Bahn (16, 17, 18) von den anderen Saugkanälen (23) getrennt ist, vorzugsweise, dass der Reserve-Saugkanal (26), gegebenenfalls jeweils ein Reserve-Saugkanal (26), im vorderen und/oder hinteren Drittel, insbesondere Viertel, des absorbierenden Kerns (4) angeordnet ist.

6. Absorbierender Kern nach einem der vorhergehenden Ansprüche, **dadurch gekennzeichnet, dass** der absorbierende Kern (4) in Längsrichtung (L) mindestens fünf, vorzugsweise genau fünf, Abschnitte mit jeweils zu den in Längsrichtung benachbarten Abschnitten variierender Anzahl an Saugkanälen (23) aufweist.

7. Absorbierender Kern nach einem der vorhergehenden Ansprüche, **dadurch gekennzeichnet, dass** der absorbierende Kern (4) entlang der Längsrichtung (L) erst drei Saugkanäle (23), dann zwei Saugkanäle (23), dann drei Saugkanäle (23) aufweist, vorzugsweise, dass der absorbierende Kern (4) entlang der Längsrichtung (L) erst einen Saugkanal (23), dann drei Saugkanäle (23), dann zwei Saugkanäle (23), dann drei Saugkanäle (23), dann einen Saugkanal (23) aufweist.

8. Absorbierender Kern nach einem der vorhergehenden Ansprüche, **dadurch gekennzeichnet, dass** die Schicht (10) mit Saugmaterial in einem Abschnitt der vorderen und/oder hinteren 40%, vorzugsweise 30%, der Länge des absorbierenden Kerns (4) mindestens drei, vorzugsweise genau drei, Saugkanäle (23) aufweist, und/oder, dass die Schicht (10) mit Saugmaterial in einem Teil der mittleren 50%, vorzugsweise der mittleren 30%, weiter vorzugsweise der mittleren 10%, und/oder in mindestens den mittleren 10%, vorzugsweise mindestens den mittleren 20%, höchstens zwei, vorzugsweise genau zwei, Saugkanäle (23) aufweist.

9. Absorbierender Kern nach einem der vorhergehenden Ansprüche, **dadurch gekennzeichnet, dass** der absorbierende Kern (4) mindestens zwei unterschiedliche Breiten aufweist, vorzugsweise, dass der absorbierende Kern (4) einen Bereich (27) von mindestens 10% der Länge des absorbierenden Kerns mit geringerer Breite und mindestens zwei, in Längsrichtung beidseits des Bereichs (27) mit geringerer Breite angeordnete, Bereiche von mindestens 15% der Länge des absorbierenden Kerns mit größerer Breite (28) aufweist, vorzugsweise, dass der Bereich (27) mit geringerer Breite die Mitte des absorbierenden Kerns (4) umfasst und/oder die Bereiche (28) mit größerer Breite die Enden in Längsrichtung (L) des absorbierenden Kerns (4) umfassen oder auf mindestens 10% der Länge des absorbierenden Kerns (4) an diese heranreichen, weiter vorzugsweise, dass jeweils ein Bereich (29) mit sich graduell verändernder Breite zwischen dem Bereich (27) mit geringerer Breite und den Bereichen (28) mit größerer Breite angeordnet ist.

10. Absorbierender Kern nach Anspruch 9, **dadurch gekennzeichnet, dass** in dem Bereich mit geringerer Breite (27) die spezifische Kapazität des absorbierenden Kerns, zumindest teilweise, vorzugsweise zu mindestens 60 %, weiter vorzugsweise zu mindestens 80%, der Fläche, weiter vorzugsweise vollständig, größer ist als in mindestens 50%, vorzugsweise mindestens 80%, weiter vorzugsweise 100%, der Fläche eines oder beider Bereiche (28) mit größerer Breite.

11. Absorbierender Kern nach einem der vorhergehenden Ansprüche, **dadurch gekennzeichnet, dass** der absorbierende Kern (4) eine Bahn (16) aufweist, die den absorbierenden Kern (4) in zwei Saugkanäle (23) teilt und die durch die mittleren 20%, vorzugsweise die mittleren 35%, der Länge des absorbierenden Kerns (4) verläuft, und die währenddessen zumindest abschnittsweise, vorzugsweise durchgehend, in den mittleren 20%, vorzugsweise den mittleren 10%, der Breite des absorbierenden Kerns (4) verläuft.

12. Absorbierender Kern nach Anspruch 11, **dadurch gekennzeichnet, dass** sich an die eine Bahn (16) im vorderen und/oder im hinteren Teil des absorbierenden Kerns (4) zwei Bahnen (17, 18, 18a) in Form auseinanderlaufender Arme, insbesondere U-förmig oder C-förmig oder mit der einen Bahn (16) T-förmig, anschließen, vorzugsweise, dass die zwei Bahnen (17, 18, 18a) im vorderen und/oder hinteren Teil des absorbierenden Kerns (4) ausgehend von der einen Bahn (16) in der Übergangszone (21) und/oder einem Bereich (29) mit sich graduell verändernder Breite beginnen und/oder enden oder sich hindurch erstrecken.

13. Absorbierender Kern nach einem der vorhergehenden Ansprüche, **dadurch gekennzeichnet, dass** die Bahnen (16, 17, 18) Kanäle sind, die zumindest in Längsrichtung (L) eine schnellere Flüssigkeitsleitung als die Saugkanäle (23) aufweisen, und/oder, dass die Bahnen (16, 17, 18) kein oder im Wesentlichen kein Saugmaterial, insbesondere keinen Superabsorber, aufweisen.

14. Hygieneartikel, insbesondere Windel, mit einem absorbierenden Kern (4) nach einem der vorhergehenden Ansprüche.

15. Herstellungsverfahren für einen absorbierenden Kern nach einem der Ansprüche 1 bis 15, **dadurch gekennzeichnet, dass** das körperseitige Deckblatt (8) oder das körperferne Rückenblatt (9) in eine mit einer Unterdruckquelle verbundene Form eingelegt wird, dass die Form mindestens einen Volumeneinsatz in Form der Bahnen (16, 17, 18) aufweist, dass auf das Deckblatt (9) oder das Rückenblatt (9) Saugmaterial aufgebracht wird, dass im Bereich des Volumeneinsatzes weniger oder kein Saugmaterial aufgebracht wird oder das Saugmaterial im Bereich des Volumeneinsatzes von der Unterdruckquelle nicht oder weniger angesogen wird und so nicht oder weniger im Bereich des Volumeneinsatzes haften bleibt, vorzugsweise, dass die Form mittels einer Bürste abgebürstet wird, wodurch die Tiefe (T) des Saugmaterials homogenisiert und der Volumeneinsatz, zumindest großteils, insbesondere vollständig, von Saugmaterial befreit wird, dass das Deckblatt (8), ggf. das Saugmaterial und das Rückenblatt (9) miteinander verbunden, insbesondere verklebt werden.
